# EUROPEAN PATENT APPLICATION

(11) **EP 2 489 728 A1**
(43) Date of publication of application: **22.08.2012**
(21) Application number: 11165578.3
(22) Date of filing: 15.06.2007
(51) Int. Cl.: C12N 5/0789

(54) **Processing procedure for peripheral blood stem cells**

(30) Priority: 15.06.2006 US 814235 P; 20.06.2006 US 815399 P; 21.08.2006 US 839311 P
(62) Divisional of application: 07809600.5
(71) Applicant: Neostem, Inc, New York, NY 10170 (US)
(72) Inventor: Rodgerson, Denis, O., Malibu, CA 90265 (US); Smith, George, S., Pacific Palisades, CA 90272 (US); Allin, Ronald, E., Woodland Hills, CA 91367 (US); Marasco, Wayne, A., Wellesley, MA 02481 (US)
(74) Representative: Pohlman, Sandra M.

(57) **Abstract**

An elective healthcare insurance model using an individual's own peripheral blood stem cells for the individual's future healthcare uses. An individual can elect to have his or her own stem cells collected, processed and preserved, while he or she is in healthy or "pre-disease" state, for future distribution for his or her healthcare needs. The process includes methods of collection, processing, preservation and distribution of adult (including pediatric) peripheral blood stem cells during non-diseased state. The stem cells collected will contain adequate dosage amounts, for one or more transplantations immediately when needed by the individual for future healthcare treatments. The collected adult or non-neonate child peripheral blood stem cells can be aliquoted into defined dosage fractions before cryopreservation so that cells can be withdrawn from storage without the necessity of thawing all of the collected cells.

## Description

**RELATED APPLICATIONS**

This application claims the benefit of priority to U.S. provisional application 60/814,235, filed June 15, 2006, U.S. provisional application 60/815,399, filed June 20, 2006, and U.S. provisional application 60/839,311, filed August 21, 2006, which are all herein incorporated by reference in their entireties.

**FIELD OF THE INVENTION**

This application relates to methods of preparing and using autologous adult stem cells obtained from non-disease or pre-disease subjects.

**BACKGROUND**

Stem cell transplantations have been used either alone (*e.g*., for congenital diseases) or in conjunction with other treatments such as chemotherapy. Stem cells are most often used because of their ability to reconstitute cells of one or more cell lineages when administered to a patient. Most stem cell transplants use either stem cells from matched donors (allogeneic) or stems cells collected from the patients (autologous) immediately before their treatment. There are number of drawbacks in allogeneic transplantations, however, such as immune rejection and graft-versus-host-disease. Allogeneic transplantation also is much more expensive than autologous transplantation.

The advantage of the autologous transfer methods is that is that the patient's own cells, including stem cells, are reintroduced into the patient. The use of the patient's own adult stem cells reduces the risk that the cells would be rejected by the immune system. Stem cells from a subject other than the patient could cause transplant rejection, and give rise to a condition called graft- versus- host disease (GVHD)in which the infused cells attack the cells of the recipient's body. These represent a significant disadvantage, as GVHD is a difficult problem that can only be circumvented with immunosuppressive drugs.

Potential sources of stem cells include embryonic stem cells, stem cells collected from umbilical cords, and adult stem cells. Unfortunately, while large numbers of embryonic stem cells are relatively easy to grow in culture, these cells are rare in mature tissues and methods for expanding their numbers in cell culture are still in development. This is an important distinction, as large numbers of cells are needed for stem cell replacement therapies. Further, embryonic stem cells from a subject introduced into a patient is allogeneic, and therefore increases the risk of transplant rejection. Stem cells collected from umbilical cords may be used for some treatments, but the low cell dose, immaturity, and incomplete complement of cells limit the immediate use of these stem cells for some treatments.

An adult stem cell, or somatic stem cell, is an undifferentiated cell found among differentiated cells in a tissue or organ. An adult stem cell can renew itself, and can differentiate to yield the major specialized cell types of the tissue or organ. The primary role of adult stem cells in a living organism is to maintain and repair the tissue in which they are found. Adult stem cells represent a source for stem cells for autologous transplantation, thereby minimizing the risk of transplant rejection. The use of the patient's own adult stem cells would mean that the cells would not be rejected by the immune system. This represents a significant advantage as immune rejection is a difficult problem that can only be circumvented with immunosuppressive drugs. There are therefore major practical and ethical advantages of using adult stem cells for autologous transfer, not the least of which is an elimination of the risk of transplant rejection.

Autologous stem cells collected from the individual right before their treatment, however, may contain contaminated or compromised cells from the disease being treated. "Compromised cells" refer to stem cells that are not as viable as those from an individual which does not have the disease or disorder. For example, stem cells from a smoker or from a patient with difficulties supplying oxygen to the body (*e.g*., emphysema) are not diseased. These stem cells are compromised, however, because (1) they are not as capable of self renewal; and (2) they are less able to generate progenitor cells or terminally-differentiated cells. Unfortunately, there is a current unmet need for an uncompromised human stem cell population sufficient for the treatment of a disease by autologous transfer. One advantage of the current invention is the ability to collect a large amount of adult stem cells and circumvent the problem described above.

Further, current methods for stem cell storage involve collection of stem cells from embryonic cord blood and the collection of stem cells from blood donations. The utility of these techniques are limited because of the small proportion of total number of stem cells in the peripheral blood and because only a limited amount of blood may be collected from a blood transfusion. A potential advantage of using stem cells from an adult is that the patient's own cells could be expanded in culture and then reintroduced into the patient. Thus, there is also an unmet need in collecting human stem cell population for long term cryogenic storage and for the eventual thawing of the cryopreserved cell population for the treatment of a disease by autologous transfer.

The embodiments described herein address various problems associated with the prior arts by providing a method and facility to collect, process, and store, and distribute healthy stem cells for future treatments of an individual's healthcare needs arise.

Throughout this description, including the foregoing description of related art, any and all publicly available documents described herein, including any and all U. S. patents, are specifically incorporated by reference herein in their entirety. The foregoing description of related art is not intended in any way as an admission that any of the documents described therein, including pending United States patent applications, are prior art to the present invention. Moreover, the description herein of any disadvantages associated with the described products, methods, and/or apparatus, is not intended to limit the invention. Indeed, aspects of the invention may include certain features of the described products, methods, and/or apparatus without suffering from their described disadvantages.

**SUMMARY**

This invention provides an elective healthcare insurance model using an individual's own peripheral blood stem cells for the individual's future healthcare uses. More specifically, this invention provides a method in which an individual can elect to have his or her own stem cells collected, processed and preserved, while he or she is in a healthy state (at a time with no immediate perceived health condition requiring treatment using his own stem cells), for future distribution for his or her healthcare needs. The invention also embodies methods of collection, processing, preservation and distribution of adult (including pediatric) peripheral blood stem cells during non-diseased state. The stem cells collected will contain adequate dosage amounts, for one or more transplantations immediately when needed by the individual for future healthcare treatments.

Thus, according to one embodiment, there is provided a method of making stem cells available to a subject, comprising the steps of: the proactively collecting the stem cells from a subject with no immediate perceived health condition requiring treatment using his own collected stem cells; collecting stem cells from the subject; at the time of collection, earmarking the collected stem cells for use by the subject; preserving the collected stem cells in storage; and retrieving the stored stem cells if and when needed by the subj ect. In preferred embodiments, the subject is a human.

According to a preferred embodiment, the stem cells may be collected by an apheresis process. Accordingly, there is provided a method for collecting autologous adult stem cells from a pre-disease human subject; collecting adult stem cells from the peripheral blood of a pre-disease human subject using an apheresis process; at the time of collection, earmarking the collected cells for use by the human subject; and preserving the collected cells to maintain the cellular integrity of the cells.

According to another preferred embodiment, there is provided a method of collecting autologous adult stem cells from a pre-disease subject comprising the steps of administering to the pre-disease subject a stem cell stem cell potentiating agent; collecting adult stem cells from peripheral blood pre-disease subject using an apheresis process; at the time of collection, earmarking the collected cells for use by the subject; and preserving the collected cells to maintain the cellular integrity of the cells.

According to yet another preferred embodiment, there is provided a method of collecting autologous adult stem cells from a pre-disease subject comprising the steps of administering to the pre-disease subject a stem cell stem cell potentiating agent; collecting adult stem cells from peripheral blood pre-disease subject using an apheresis process; at the time of collection, earmarking the collected cells for use by the subject; and preserving the collected cells to maintain the cellular integrity of the cells; wherein the pre-disease subject is administered a stem cell potentiating agent on two consecutive days, with the subject receiving one dose per day, and wherein the apheresis process is performed on the third consecutive day.

According to another preferred embodiment, there is provided a method of collecting autologous adult stem cells from a pre-disease subject comprising the steps of: administering to the pre-disease subject at least two doses of G-CSF of about 1 µg/kg/day to 8 µg/kg/day; collecting adult stem cells from peripheral blood pre-disease subject using an apheresis process; at the time of collection, earmarking the collected cells for use by the subject; and preserving the collected cells to maintain the cellular integrity of the cells. The pre-disease subject may be administered at least two doses of G-CSF within a 2 to 6 day period. Preferably, at least two doses of G-CSF is administered on two consecutive days, with the subject receiving only one dose per day. More preferably, the subject receives two doses of G-CSF administered on consecutive days. In another preferred embodiment, the pre-disease subject is administered at least two doses of G-CSF within about 12 to about 36 hours of each other.

Accordingly to another preferred embodiment, the G-CSF is administered to a subject at a dose of about 4 to about 6 µg/kg/day or equivalent thereof.

Accordingly to another preferred embodiment, about 50 µg to about 800 µg per dose of G-CSF is administered subcutaneously to the subject.

Accordingly to another preferred embodiment, about 300 µg to about 500 µg per dose of G-CSF is administered subcutaneously to the subject.

Accordingly to another preferred embodiment, the subject is a human subject that has met at least one condition selected from the group consisting of between 10 and 200 kg in weight and between 2 to 80 years old.

The G-CSF may be administered subcutaneously. Preferably, about 480 µg per dose of G-CSF is administered subcutaneously to the pre-disease subject.

The collection of adult stem cells from peripheral blood using an apheresis process may be conducted the day after the second dose of G-CSF is administered. In a preferred embodiment, the collection of adult stem cells from peripheral blood using an apheresis process is conducted about 12 to about 36 hours after the second dose of G-CSF is administered. According to one embodiment, the collecting step is conducted when the subject is an adult or a non-neonate. According to another embodiment, the collecting step includes the step of collecting at least on the order of greater than 200 X 10⁸ total nucleated cells per subject in a single collection process. Preferably, the collecting step includes the step of collecting at least on the order of greater than 250 X 10⁸ total nucleated cells per subject in a single collection process.

According to another embodiment, the collecting step is undertaken over multiple sessions.

According to yet another embodiment, the preserving step comprises storing the collected cells in a stem cell bank.

According to another preferred embodiment, there is provided a process of stem cell banking comprising the steps of: (a) administrating one or more stem cell potentiating agents to a person to increase the amount of stem cells in the peripheral blood of the person; (b) collecting at least one population of stem cells and at least one population of non-stem cells from peripheral blood of the person using an apheresis process, wherein the person has no immediate perceived health condition requiring treatment using his own collected stem cells; (c) preserving the at least one population of stem cells and the at least one population of non-stem cells as a preserved populations of cells; (d) retrieving the preserved populations of cells for autologous transplantation of the at least one population of stem cells and at least one population of non-stem cells into the person. Preferably, the one or more stem cell potentiating agents is selected from the group consisting of G-CSF, GM-CSF, dexamethazone, a CXCR4 receptors inhibitor and a combination thereof. The CXCR4 receptor inhibitor may be selected from the group consisting of AMD3100, ALX40-4C, T22, T134, T140, and TAK-779.

According to another embodiment, the process is performed without HLA typing of the one population of stem cells or the at least one population of non-stem cells

According to another embodiment, administration is performed for at least one week before the collecting step.

According to yet another embodiment, the health condition is selected from the group consisting of a neoplastic disorder, an immune disorder, and leucopenia.

According to yet another embodiment, the collecting step is conducted when the person is an adult or a non-neonate child. The collecting step may be performed at least two times, at least three times, or at least five times. According to preferred embodiments, the collecting step collects at least 1 x 10⁶ total nucleated cells per kilogram weight of the person in a single collection session; at least 2 x 10⁶ total nucleated cells per kilogram weight of the person in one or more collection session; at least 3 x 10⁶ total nucleated cells per kilogram weight of the person in one or more collection session; or at least 5 x 10⁶ total nucleated cells per kilogram weight of the person in one or more collection session.

According to preferred embodiments, the apheresis process is performed for at least one hour in the collecting step; at least two hours in the collecting step; at least three hours in the collecting step; at least four hours in the collecting step.

According to yet another embodiment, the apheresis process releases additional cells into the peripheral blood of the person. The additional cells may be selected from the group of stem cells, progenitor cells, and terminally differentiated cells.

According to yet another embodiment, the preserving step preserves cells collected in the collecting step before substantial cell divisions.

According to yet another embodiment, the preserving step may also comprise the step of processing the stem cells into multiple separate containers for storage. The processing step may also comprise the step of isolating one cell population enriched or depleted for a stem cell surface antigen. The stem cell surface antigen may be selected from the group consisting of CD34, KDR, CD45, and CD 133.

According to yet another embodiment, the processing step may also comprise the step of isolating one cell population enriched or depleted for a progenitor cell surface antigen. The progenitor cell surface antigen may be selected from the group consisting of CD45, Lin, Muc-18, CK19, Nestin, and KDR.

According to yet another embodiment, the processing step may also comprise the step of isolating one cell population enriched for one or more terminally differentiated cell surface antigen. The one or more terminally differentiated cell surface antigen may be selected from the group consisting of CD-4, CD-8, Flk1, myosin, bone specific alkaline phosphatase, osteocalcin, bone morphogenic protein receptor, CD38, CD44, Thy-1, and adipocyte lipid binding protein.

According to yet another embodiment, the processing step may also comprise the step of analyzing at least, one characteristic of one cell in the one population of stem cells or at least one population of non-stem cells. The at least one characteristic may be a DNA or RNA sequence of the cell, or may be a proteome of the cell.

According to yet another embodiment, the processing step may also involve treating the one population of stem cells or the at least one population of non-stem cells with an agent to enhance the storage, viability, or therapeutic ability of the cell population. The treating may involve transforming the one population of stem cells or the at least one population of non-stem cells with a nucleic acid.
According to yet another embodiment, the preserving step may also comprise the step of determining from the collected population of cells at least a distinctive property associated with the person prior to storing in a the stem cell bank, so as to provide a means of secured identification to match the collected stem cells with the person at the time of use. The distinctive property may be a DNA or RNA sequence, or may be a proteome of a cell the one population of stem cells or the at least one population of non-stem cells. The determining step may further include providing an indicia with each population of cells representing information of the distinctive property The indicia may be embodied in at least one of a label, bar code, magnetic strip, and microchip, or may be embedded within the preserved collected populations of cells.

According to yet another embodiment, the preserving step may also comprise cryopreservation of the at least one population of stem cells and at least one population of non-stem cells. The at least one population of stem cells and at least one population of non-stem cells may cryopreserved in separate containers or may be cryopreserved in the same container.

According to yet another embodiment, the process may further comprise the step of: (f) administering the stem cell to the person in an autologous transfer. This process may be used to treat a person in a leukopenic state. Additionally, the autologous transfer may be performed without HLA typing.

According to yet another preferred embodiment, there is provided a method of phenotypically characterizing mobilized peripheral blood stem cells as a diverse population of stem cells and progenitor cells for their ability to become committed linear specific progenitor cells.

According to other preferred embodiments, compositions and methods are provided for treating a patient in need thereof comprising administering to a subject an autologous mixture of stem cells, progenitor cells, and optionally functional cells. For example, the present invention is useful to enhance the effectiveness of hematopoietic progenitor cell engraftment as a treatment for cancer.

### Brief Description Of The Drawings

Figure 1 is a flow diagram schematically representing the inventive process in accordance with one embodiment of the present invention.

Figure 2 is a flow diagram schematically representing the stem cell collection process in accordance with one embodiment of the present invention.

Figure 3 is a flow diagram schematically representing stem cell processing in accordance with one embodiment of the present invention.

Figure 4 is a flow diagram schematically representing the procurement, collection, and processing processes of a preferred embodiment.

Figure 5 is a flow diagram schematically representing the stem cell collection process in accordance with a preferred embodiment of the present invention.

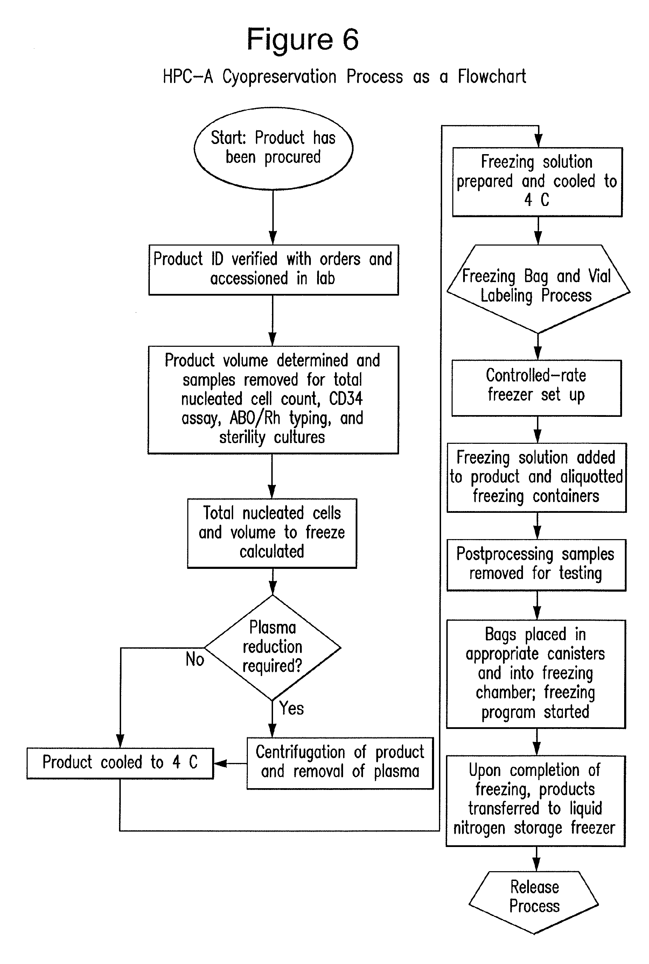

Figure 6 is a flow diagram schematically representing the stem cell cyropreservation process in accordance with one embodiment of the present invention.

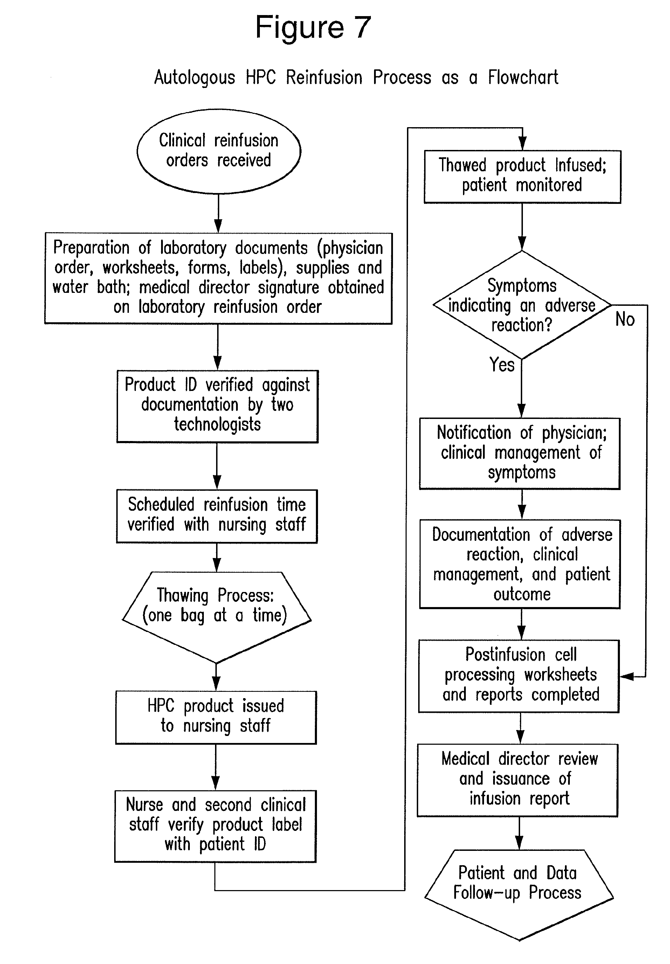

Figure 7 is a flow diagram schematically representing the autologous stem cell reinfusion process in accordance with one embodiment of the present invention.

**DETAILED DESCRIPTION OF THE PREFERRED EMBODIMENTS**

This description is made for the purpose of illustrating the general principles of the invention and should not be taken in a limiting sense. The scope of the invention is best determined by reference to the appended claims. This invention has been described herein in reference to various embodiments and drawings. It will be appreciated by those skilled in the art that variations and improvements may be accomplished in view of these teachings without deviating from the scope and spirit of the invention.

For the purposes of promoting an understanding of the embodiments described herein, reference will be made to preferred embodiments and specific language will be used to describe the same. The terminology used herein is for the purpose of describing particular embodiments only, and is not intended to limit the scope of the present invention. As used throughout this disclosure, the singular forms "a," "an," and "the" include plural reference unless the context clearly dictates otherwise. Thus, for example, a reference to "a composition" includes a plurality of such compositions, as well as a single composition, and a reference to "a therapeutic agent" is a reference to one or more therapeutic and/or pharmaceutical agents and equivalents thereof known to those skilled in the art, and so forth. Also, for ease of discussion, the pronoun "he" is used. It is to be understood that the term "he" includes "he" and/or "she".

Hematopoietic reconstitution is an established therapy in a variety of diseases and disorders such as anemias; malignancies; immune and autoimmune deficiencies, disorders and dysfunctions; and neurological disease such as Parkinson's disease, amyotrophic lateral sclerosis (ALS, also known as "Lou Gehrig's disease") and other neurological disorders. Peripheral blood, however, is not generally known to comprise many stem cells. Surprisingly, using the methods of the invention, peripheral blood can be an especially rich source of adult stem cells.

Hematopoietic reconstitution with autologous peripheral blood stem cells can occur with or without ablation of the bone marrow. Ablation is the partial or complete destruction of a cell system by chemotherapy, ionizing radiation or a combination of chemotherapy and ionizing radiation. Such ablation can also occur as result of exposure to the ionizing radiation that is produced following a nuclear explosion. While ablation of the hematopoietic system is the most common, other forms of cell ablation, such as immunoablation by high-dose cyclophosphamide is also possible. Other methods of ablation include, for example, selective T-cell ablation with bismuth-213-labeled anti-T cell receptor antibody.

In the hematopoietic system, pluripotent stem cells are believed to be able to repopulate all of the blood cell lineages in an ablated mammal. Mammalian blood cells provide for an extraordinarily diverse range of activities. The blood cells are divided into several lineages, including lymphoid, myeloid and erythroid. The lymphoid lineage, comprising B cells and T cells, provides for the production of antibodies, regulation of the cellular immune system, detection of foreign agents in the blood, detection of cells foreign to the host, and the like. The myeloid lineage, which includes monocytes, granulocytes, megakaryocytes as well as other cells, monitors for the presence of foreign bodies in the blood stream, provides protection against neoplastic cells, scavenges foreign materials in the blood stream, produces platelets, and the like. The erythroid lineage provides the red blood cells, which act as oxygen carriers.

It should be understood that there is a distinction between a "hematopoietic stem cells" or "hematopoietic pluripotent stem cell" and a "stem cell collected from the hematopoietic system." A "hematopoietic stem cells" or "hematopoietic pluripotent stem cell" is a stem cell that by differentiation, and division, can repopulate the various lineages of the hematopoietic system. Hematopoietic stem cells (HSC) are stem cells and the early precursor cells which give rise to all the blood cell types that include both the myeloid (monocytes and macrophages, neutrophils, basophils, eosinophils, erythrocytes, megakaryocytes/platelets and some dendritic cells) and lymphoid lineages (T-cells, B-cells, NK-cells, some dendritic cells). A hematopoietic pluripotent stem cell does not have to be collected from the hematopoietic system. For example, hematopoietic stem cells may be collected from gut, spleen, kidney or ovaries - tissues that are not part of the hematopoietic system.

Conversely, a "stem cell collected from the hematopoietic system," such as the "peripheral blood stem cells" or "PBSC" collected by an apheresis process of this disclosure may be a stem cell for all tissue types in the body. That is, a stem cell collected by apheresis process of this invention may be any stem cell, such as a neural stem cell, an adipose tissue stem cell, a liver stem cell, a muscle stem cell, or a hematopoietic stem cell, etc. Thus, a stem cell collected by the method of this disclosure may give rise to any lineage of cells in a mammalian body, such as, for example, a neural stem cell, an adipose tissue stem cell, a liver stem cell, a muscle stem cell, or a hematopoietic stem cell etc.

As used in the context of the present invention described herein, the term "donor" or "subject" refers to a person or a animal from whom stem cells are collected. The stem cells may be used in an autologous transfer for future treatment of that same donor or subject. The terms "donor" or "subject" refers to all animals, in particular vertebrates, for which the collection of peripheral blood is possible. Examples of such vertebrates are mammals which includes human and commercially valuable livestock and research animals such as horses, cows, goats, rats, mice, rabbits, pigs, and the like. An example of such a mammal is a human such as a human infant, child, or adult. For ease of discussion in this patent application, we use a human being (identified as a person, a patient or a donor) as a non-limiting example of such an animal. According to preferred embodiments, the subject is a human.

An important aspect of the invention relates to a process of making stem cells available to a subject, comprising the steps of: the proactively collecting the stem cells from a subject with no immediate perceived health condition requiring treatment using his own collected stem cells; collecting stem cells from the subject; at the time of collection, earmarking the collected stem cells for use by the subject; preserving the collected stem cells in storage; and retrieving the stored stem cells if and when needed by the subject.

In preferred embodiments, the subject is a human. Accordingly, an important aspect of the invention relates to a process of making stem cells available to a person, comprising the steps of: the person proactively electing to have his stem cells collected with no immediate perceived health condition requiring treatment using his own collected stem cells; collecting stem cells from the person; at the time of collection, earmarking the collected stem cells for use by the person; preserving the collected stem cells in storage; and retrieving the stored stem cells if and when needed by the person.

Preferably, the subject or person is in a non-disease or pre-disease state. It should be noted that the term "pre-disease" state (versus "post-disease" state) as used herein covers the absolute term of "healthy", "no disease" (versus "not healthy/diseased") and a relative term of a gradation in the disease progression ("healthier than" or "less diseased" than post-disease state). Since "pre-disease" can be defined by a time prior to a subject being diagnosed with a disease, the subject could be healthy in an absolute term or might already have the disease where the disease has not yet manifested itself, not yet been diagnosed, or not yet detected. Even in the latter scenario, for such a "pre-disease" state, it is possible that the disease may not be so widespread such that it has reached the cells collected; or even if the cells collected are diseased, they may be less aggressive or are of a healthier grade due to the early stage of their development, or the cells still retain some functioning necessary to combat the same disease and/or other diseases. Thus, the term "healthy" cells covers both the absolute term that the cells are healthy, and the term that, relatively speaking, these collected cells (from the subject before he becomes a patient) are healthier than what the patient (in his "post-disease" state) currently have in his body.

Specifically, "pre-disease" state could refer to prior diagnosis or knowledge of a specific targeted disease or diseases, or class or classes of diseases, of the subject (collectively "specific diseases"), such that stem cell can be collected from the subject at an opportune time in anticipation of the subject manifesting the specific diseases in the future. For example, in view of family health history, genetic history and/or profiling, a subject may be deemed to have a certain probability of contracting a certain specific disease (*e.g*., a certain cancer) during adult years.

Other definitions of "pre-disease" state may be adopted without departing from the scope and spirit of the present invention. For example, certain standards may be established to pre-diagnose the stem cell subject as being in a "pre-disease" state. This type of pre-diagnosis may be established as an optional screening process prior to collection of stem cells from the subject in the "pre-disease" state. Such "pre-disease" state standards may include one or more of the following considerations or references prior to collection, such as (a) pre-specific disease; (b) prior to actual knowledge by subject and/or health professionals of specific or general diseases; (c) prior to contraction and/or diagnosis of one or more classes of diseases; (d) prior to one or more threshold parameters of the subject relating to certain diseases, for example at a certain age, with respect to certain physical conditions and/or symptoms, with respect to certain specific diseases, with respect to certain prior treatment history and/or preventive treatment, etc.; (e) whether the subject fits into one or more established statistical and/or demographic models or profiles (*e.g.*, statistically unlikely to acquire certain diseases); and (f) whether the subject is in a certain acceptable health condition as perceived based on prevailing medical practices.

The present invention provides an elective healthcare insurance model using an individual's own peripheral blood stem cells for the individual's future healthcare uses. More specifically, this invention provides a method in which an individual can elect to have his or her own stem cells collected, processed and preserved, while he or she is in healthy state, for future distribution for his or her healthcare needs. The invention also embodies methods of collection, processing, preservation, and distribution of adult (including pediatric) peripheral blood stem cells during non-diseased state. The stem cells collected will contain adequate dosage amounts, for one or more transplantations immediately when needed by the individual for future healthcare treatments.

In accordance with the process provided by the present invention, stem cells are collected from the subject during his early years before the disease manifests itself, which stem cells are banked in anticipation of this specific disease in the future. The donor may be subject to a medical examination to confirm that he is "pre-disease" with respect to the specific disease. At the time of stem cell collection, the subject may be diagnosed to have a disease or diseases, or class or classes of diseases different from the specific disease, which diagnosed diseases may be acceptable with respect to stem cell collection and/or the specific disease as perceived by prevailing medical practices. In other words, stem cells may be collected at a stage when the subject may actually possess, or be diagnosed with, a health condition that is not similar to the disease to be treated by stem cell transplantation.

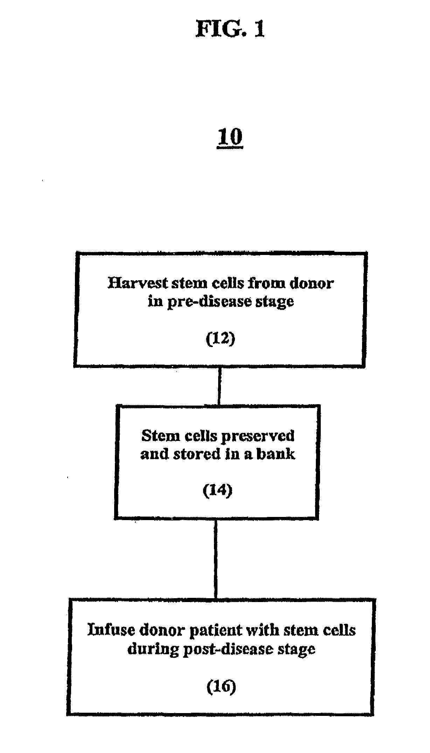

Referring to Figure 1, the inventive process 10 in accordance with one embodiment of the present invention broadly comprises the following steps:

(1) Healthy stem cells are harvested from a subject in the "pre-disease" stage (12). The term "pre-disease" means indicate the state in which the subject is healthy, or before the subject has developed, manifested, or been diagnosed with any or a particular disease, which is known to affect the quality of the stem cells, or before the subject is deemed to be in a health condition that would render the subject not to be in a state qualified for stem cell collection.

(2) The pre-diseased cells may be preserved and stored in a bank (14), *e.g*., by cryopreservation, for many years, such as for 500 years or less. Preferably, the pre-diseased cells are preserved and stored in a bank throughout a subjects lifetime, after which the cells may be discarded or made available for allogeneic transplantation or other use. The pre-diseased cells may be preserved and stored in a bank until such time that the subject is in need of a stem cell transplantation. Thus, accordingly to preferred embodiments, pre-diseased cells are preserved and stored in a bank for at least 0 to about 100 years, for about 1 year to about 100 years, for about 5 years to about 80 years, for about 5 years to about 50 years, for about 10 years to about 30 years, for about 10 years to about 20 years, for about 15 years to about 30 years, etc.

(3) In the event that the subject develops, manifests, or is diagnosed with a disease ("post-disease" state), he is infused with the above previously preserved pre-disease cells (16). The term "post-disease" denotes the state at or after which the subject develops, has developed, manifests, has manifested, has been diagnosed with a disease, or his disease has become detectable or been detected.

Preferably, the disease is a cancer or other disease where treatment is benefited by the restoration of hematopoietic cells levels in a subject. According to another preferred embodiment, there is provided a method for collecting autologous adult stem cells from a pre-cancer subject comprising: collecting adult stem cells from peripheral blood pre-cancer subject using an apheresis process; at the time of collection, earmarking the collected cells for use by the human subject; and preserving the collected cells to maintain the cellular integrity of the cells. As used herein the term "pre-cancer" refers to the state of a subj ect prior to the diagnosis of cancer, and therefore the subject may be healthy in an absolute term or might already have the disease but only that it has not manifested itself through diagnoses or detection. Likewise, the term "post-cancer" refers to the state of a subject after the subject has been diagnosed with cancer requiring treatment.

Various stages of the inventive process are discussed in greater detail below.

***Stem Cell Collection Process***

An inventive aspect of the stem cell collection process is directed to the timing and the health state of the individual when the collection occurs. According to preferred embodiments, the collection process occurs when the individual is in a non-diseased or pre-disease state. The stem cells may be collected during a pre-disease stage in which the person may be diagnosed with a health condition that is not similar to the disease to which the collected stem cells are intended to be applied for treatment. Stem cells are primarily found in the insides of long bones (legs, hips, sternum etc.) and comprise the "bone marrow". These stem cells may leave the bone marrow and circulate in the blood stream. The physical steps of collecting stem cells may comprise those steps known in the art. Stem cells comprise approximately 0.1-1.0% of the total nucleated cells as measured by the surrogate CD34+ cells.

According to another preferred embodiment, there is provided a method for collecting an adequate stem cell dosage from an individual donor during non-diseased state, processing the stem cells collected, cryogenically preserving them for future distribution for the donor's healthcare needs. In one embodiment of the current invention, stem cells and progenitor cells are collected during the non-disease or pre-disease phase by the process of apheresis from adult or pediatric peripheral blood, processed to optimize the quantity and quality of the collected stem cells, cryogenically preserved, and used for autologous therapeutic purposes when needed after they have been thawed. Autologous therapeutic purposes are those in which the cells collected from the donor are infused into that donor at a later time.

According to a preferred embodiment, the stem cells may be collected by an apheresis process, which typically utilizes an apheresis instrument. The apheresis instrument looks very much like a dialysis machine, but differs in that it is a centrifuge while a dialysis machine uses filtration technology. Stem cell collection can be accomplished in the privacy of the donors own home or in a collection center. Blood is drawn from one arm then enters the apheresis instrument where the stem cells are separated and collected. The rest of the whole blood is then returned to the donor. A registered nurse (RN) places a needle into both arms of the subject in the same manner as a routine blood collection. The RN then operates the apheresis instrument that separates the blood elements (red cells, white cells, plasma) collecting the stem cells and returning the rest of the whole blood to the donor. The collection of stem cells requires approximately 2-4 hours during which the subject is relaxing and watching a movie. Shortly after the apheresis collection, the bone marrow releases more stem cells into the blood stream to replace the harvested stem cells. The amount of stem cells collected is a very small fraction of a person's stem cells. In a healthy individual, the stem cells can rapidly multiply and replace the lost stem cells. Thus, the procedures of the invention does not deplete the body of stem cells. Many hundreds of thousands of apheresis collections take place each year for platelets, red cells, plasma and stem cells. It has been shown to be safe and effective technology.

According to a preferred embodiment, there is provided a method for collecting autologous adult stem cells from a pre-disease human subject; collecting adult stem cells from peripheral blood pre-disease human subject using an apheresis process; at the time of collection, earmarking the collected cells for use by the human subject; and preserving the collected cells to maintain the cellular integrity of the cells. The human subject may be an adult human or non-neonate child. Accordingly, the above processes may further include the collection of adult or non-neonate child peripheral blood stem cells where the cells are then aliquoted into defined dosage fractions before cryopreservation so that cells can be withdrawn from storage without the necessity of thawing all of the collected cells.

Collection may be performed on any person, including adult or a non-neonate child. Furthermore, collection may involve one or more collecting steps or collecting periods. For example, collection (*e.g*., using an apheresis process) may be performed at least two times, at least three times, or at least 5 times on a person. During each collecting step, the number of total nucleated cells collected per kilogram weight of the person may be one million (1 x 10⁶) or more, two million or more, three million or more, or 5 million or more.

Adverse medical reactions during apheresis collections are uncommon and may consist of a tingling sensation in the mouth and fingers. This reaction is usually mild in nature and does not stop the stem cell collection.

We have found, surprisingly, that as the apheresis length increase, the number of stem cells collected increase at a rate that is greater than linear. That is, for example, a two hour apheresis process collects more than twofold the number of cells (stem cells, progenitor cells, or terminally differentiated cells) collected in a one hour apheresis process. Without being limited to a theory, it is believed that by using the methods of the invention, the apheresis process causes the release of additional cells (stem cells, progenitor cells, or terminally differentiated cells) into the peripheral blood. Thus, the apheresis process in any of the methods of the invention may be for at least one hour, at least two hours, at least three hours, at least four hours.

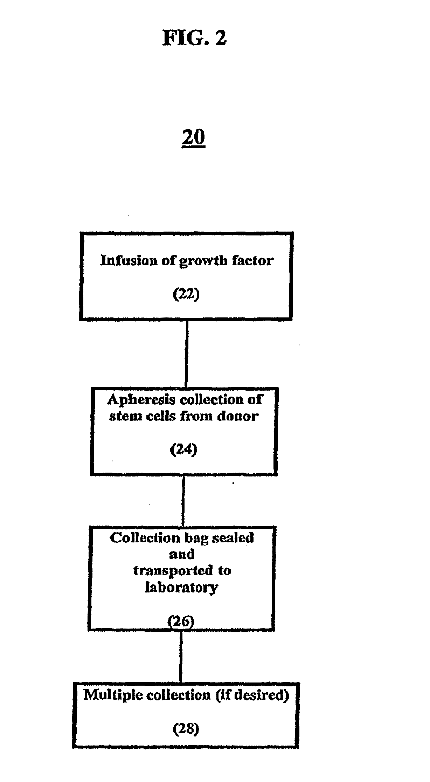

Following collection of the stem cells (26) the collection bag is sealed and then transported to the laboratory for processing, testing and cryopreservation. See Figure 2. Stem cells may be transported by methods known in the art. For example, conventional containers for blood can be used for transport, *e.g.* thermally validated containers can be transported by express methods or messengers. In these embodiments, the temperature of the container remains essentially constant over long periods of time.

Depending on the situation and the quantity and quality of stem cells to be collected from the donor, it may be preferable to collect the stem cells from donors when they are at an "adult" or a "matured" age (the term "adult" as used herein refers to and includes adult and non-neonate, unless otherwise used in a particular context to take a different meaning) and/or at a certain minimum weight. For example, stem cells are collected when the subject is within a range from 10 to 200 kg in accordance with one embodiment of the present invention, or any range within such range, such as 20 to 40 kg In addition or in the alternative, it may be required that the subject be of a certain age, within a range from 2-80 years old in accordance with one embodiment of the present invention, or any range within such range, such as 9 to 18 years old, or 12 to 16 years old, or any range of ages within such age ranges, or as determined statistically. Certain legal requirements may also proscribe and/or limit the appropriate age and/or or weight of the subject for stem cell collection.

In one embodiment of the present invention, a subject may elect to have stem cell collection in multiple stages (28), to increase the amount of stem cells to be bank for future use. See Figure 2. For example, he may elect to have stem cells collected at different age and/or weight. Different units of stem cells can be collected at each collection, as appropriate depending on the age and/or weight of the subject at the time of collection. Generally, more stem cells can be collected during a single collection process, as the age and/or weight of the subject increase. Further, in addition or in the alternative, he may elect to have stem cell collected pre-disease and post-disease (i.e., after the period of pre-disease as defined herein). Still further, in addition or in the alternative, he may elect to have stem cells collected periodically or at specified times pre-disease, independent of his weight and/or age, and to map the progress of the health condition of the donor.

***Stem Cell Potentiating Agent***

Figure 2 schematically illustrates the steps involved in the stem cell collection process (20) in accordance with one embodiment of the present invention. The amount of stem cells circulating in the peripheral blood cell may be increased with the infusion of cell growth factors prior to collection (22), such as, for example, granulocyte colony stimulating factor (G-CSF). The infusion of growth factors is routinely given to bone marrow and peripheral blood donors and has not been associated with any long lasting untoward effects. Adverse side effects are not common but include the possibility of pain in the long bones, sternum, and pelvis, mild headache, mild nausea and a transient elevation in temperature. The growth factor is given 1-6 days before peripheral blood stem cells are collected. 1-6 days after G-SCF is infused the peripheral blood stem cells are sterilely collected by an apheresis instrument (24).

In a preferred embodiment, there is provided a method of mobilizing a significant number of peripheral blood stem cells comprising the administration of a stem cell potentiating agent. The function of the stem cell potentiating agent is to increase the number or quality of the stem cells that can be collected from the person. These agents include, but are not limited to, G-CSF, GM-CSF, dexamethazone, a CXCR4 receptors inhibitor, Interleukin-1 (IL-1), Interleukin-3 (IL-3), Interleukin-8 (IL-8), PIXY-321 (GM-CSF/IL-3 fusion protein), macrophage inflammatory protein, stem cell factor, thrombopoietin and growth related oncogene, as single agents or in combination. In a preferred embodiment, there is provided a method of mobilizing a significant number of peripheral blood stem cells comprising the administration of G-CSF to a predisease subject.

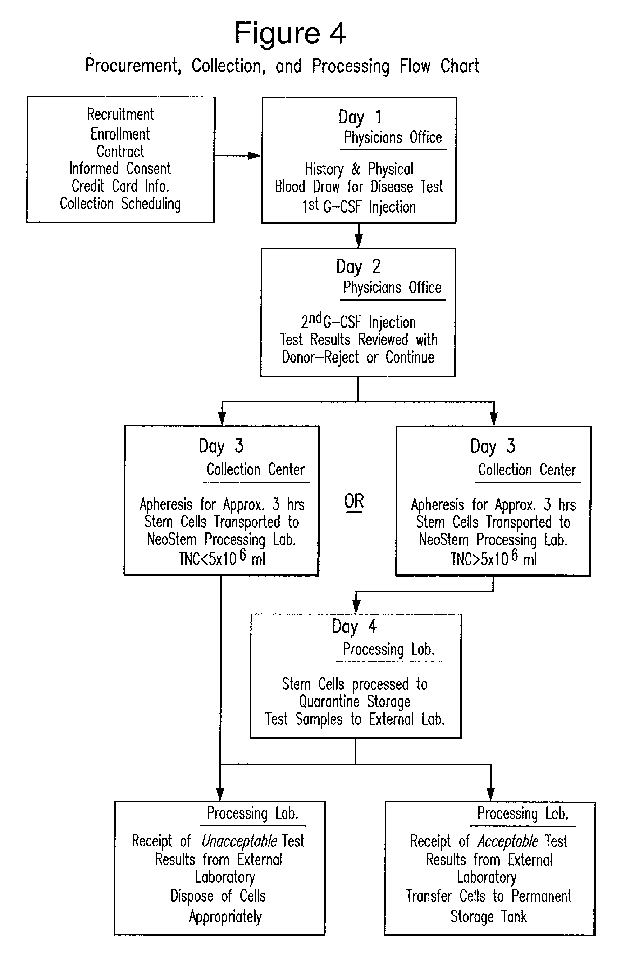

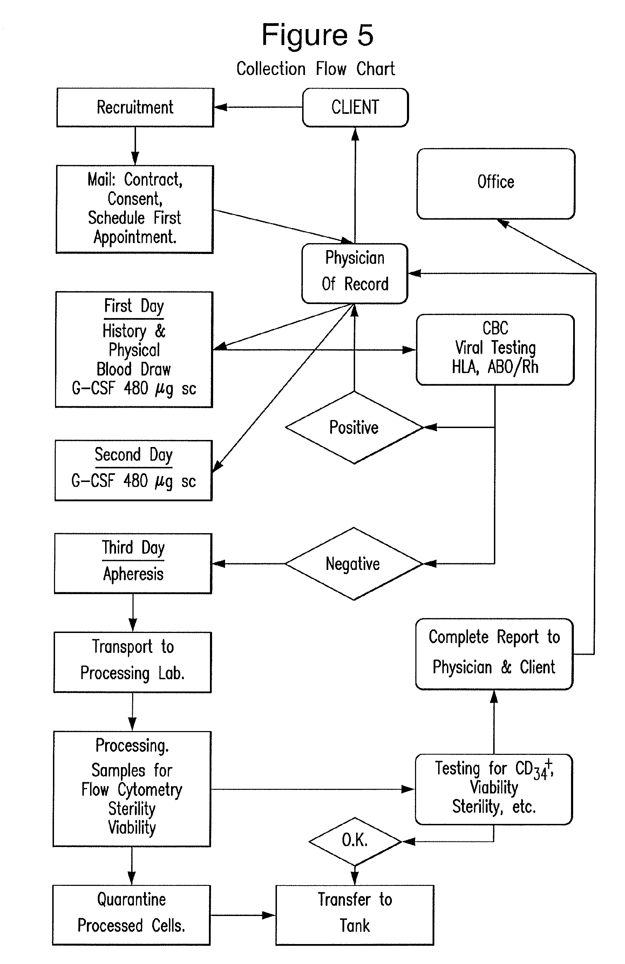

According to a preferred embodiment, the G-CSF is administered to a predisease subject over a 1 to 6 day course, which ends upon apheresis of the subjects peripheral blood. Preferably, the G-CSF is administered to a predisease subject at least twice over a 2 to 6 day period. For example, G-CSF may be administered on day 1 and day 3 or may be administered on day 1, day 3, and day 5 or, alternatively, day 1, day 2, and day 5. Most preferably, G-CSF is administered to a predisease subject twice for consecutive days over a 3 day course. Thus, according to the preferred embodiment, G-CSF is administered to a predisease subject on day 1 and day 2 followed by apheresis on day 3. See, for example, Figures 4 and 5.

Additionally, according to preferred embodiments, a low dose G-CSF is administered to a subject. Thus, a subject may receive a dose of G-CSF of about 1 µg/kg/day to 8 µg/kg/day. Preferably, G-CSF is administered to a subject at a dose of about 2 to about 7 µg/kg/day or equivalent thereof. More preferably, G-CSF is administered to a subject at a dose of about 4 to about 6 µg/kg/day or equivalent thereof. For subcutaneous injections, the dose of G-CSF may be from about 50 µg to about 800 µg, preferably from about 100 µg to about 600 µg, more preferably from about 250 µg to 500 µg, and most preferably from about 300 µg to about 500 µg.

Accordingly to another preferred embodiment, antagonist or inhibitors of CXCR4 receptors may be used as a stem cell potentiating agents. Examples of CXCR4 inhibitors that have been found to increase the amount of stem cells in the peripheral blood include, but are not limited to, AMD3100, ALX40-4C, T22, T134, T140 and TAK-779. See also, U.S. Patent No. 7,169,750, incorporated herein by reference in its entirety. These stem cell potentiating agents may be administered to the person before the collecting step. For example, the potentiating agent may be administered at least one day, at least three days, or at least one week before the collecting step. Preferably, the CXCR4 inhibitors are administered to a predisease subject at least twice over a 2 to 6 day period. For example, the CXCR4 inhibitors may be administered on day 1 and day 3 or may be administered on day 1, day 3, and day 5 or, alternatively, day 1, day 2, and day 5. Most preferably, the CXCR4 inhibitors are administered to a predisease subject twice for consecutive days over a 3 day course. Thus, according to the preferred embodiment, the CXCR4 inhibitors are administered to a predisease subject on day 1 and day 2 followed by apheresis on day 3.

The formulation and route of administration chosen will be tailored to the individual subject, the nature of the condition to be treated in the subject, and generally, the judgment of the attending practitioner. Suitable dosage ranges for CXCR4 inhibitors vary according to these considerations, but in general, the compounds are administered in the range of about 0.1 µg/kg to 5 mg/kg of body weight; preferably the range is about 1 µg/kg to 300 µg/kg of body weight; more preferably about 10 µg/kg to 100 µg/kg of body weight. For a typical 70-kg human subject, thus, the dosage range is from about 0.7 µg to 350 mg; preferably about 700 µg to 21 mg; most preferably about 700 µg to 7 mg. Dosages may be higher when the compounds are administered orally or transdermally as compared to, for example, i.v. administration.

***Stem Cell Processing***

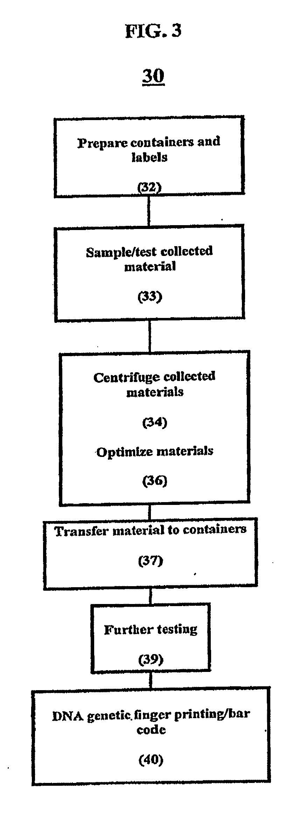

In some embodiments of the invention, after collection, the stem cells are processed according to methods known in the art (see, for example, Lasky, L. C. and Warkentin, P. I.; Marrow and Stem Cell Processing for Transplantation; American Association of Blood Banks (2002)). In an embodiment of the invention schematically illustrated in Figure 3, processing (30) may include the following steps: preparation of containers (*e.g*., tubes) and labels (32), sampling and/or testing of the collected material (33), centrifugation (34), transfer of material from collection containers to storage containers (37), the addition of cryoprotectant (38), etc. In some embodiments, after processing, some of the processed stem cells can be made available for further testing (39).

The cells also may be processed, preferably before the preservation step is conducted. Processing may involve, for example, enrichment or depletion of cells with certain cell surface markers. Any cell surface marker, including the cell surface markers listed anywhere in this specification may be used as a criteria for enrichment or depletion. Furthermore, processing may involve analyzing at least one characteristic of one cell in the one population of stem cells or the at least one population of non-stem cells. The characteristic may be a DNA or RNA sequence. For example, the genomic DNA or RNA may be partially or completely sequenced (determined). Alternatively, specific regions of the DNA or RNA of a cell may be sequenced. For example, nucleic acids from a cell or a cell population may be extracted. Specific regions of these nucleic acid may be amplified using amplification probes in an amplification process. The amplification process may be, for example PCR or LCR. After amplification, the amplimers (products of amplification) may be sequenced. Furthermore, the DNA and RNA may be analyzed using gene chips, using hybridization or other technologies.

Specific uniqueness of this invention is that there will be no requirement for any kind of tissue typing since the collected stem cells will be used for autologous transplantation. However, tissue typing of specific kinds may be used for sample identification or for the use of these stem cells for possible allogeneic use. This type of information may include genotypic or phenotypic information. Phenotypic information may include any observable or measurable characteristic, either at a macroscopic or system level or microscopic, cellular or molecular level. Genotypic information may refer to a specific genetic composition of a specific individual organism, including one or more variations or mutations in the genetic composition of the individual's genome and the possible relationship of that genetic composition to disease. An example of this genotypic information is the genetic "fingerprint" and the Human Leukocyte Antigen (HLA) type of the donor. In some embodiments of the invention the stem cells will be processed in such a way that defined dosages for transplantation will be identified and aliquoted into appropriate containers.

In preferred embodiments, the number of cells collected in a single collection session may be equal or greater than 2x10¹⁰ total nucleated cells, or at least on the order of 10⁹, or 10⁸, or 10⁷, or 10⁶, or 10⁵ total nucleated cells, depending on the weight and age of the donor. Aliquoting of these cells may be performed so that a quantity of cells sufficient for one transplant (1x10⁹ total nucleated cells) will be stored in one cryocyte bag or tube, while quantities of cells appropriate for micro-transplantation (supplemental stem cell infusion), will be stored in 20 aliquots of 1x10⁸ total nucleated cells, in appropriate containers (cryocyte bags or cryotubes). Generally, at least one unit is collected at each collection session, and each unit collected is targeted at more than on the order of 10⁶ total nucleated cells per kg weight of the person, in accordance with one embodiment of the present invention. This process constitutes a unique process for "unitized storage" enabling individuals to withdraw quantities of cells for autologous use without the necessity of thawing the total volume of cells in storage (further details discussed below). This may include processing the harvested stem cells (36) to optimize the quantity of total nucleated cells to ensure sufficient number of cells for targeted diseases without or with little waste of cells (i.e., disease directed dosage). Fault tolerant and redundant computer systems will be used for data processing, to maintaining records relating to subject information and to ensure rapid and efficient retrieval stem cells from the storage repositories.

The processing step may comprise the step of optimizing at least one of the number of units and the number of stem cells in each unit prior to storing in the stem cell bank, with consideration of intended disease or diseases to which the stem cells will be applied. In some cases, each unit comprises more than on the order of 10⁶ total nucleated cells per Kg weight of the person. Further, each unit may comprise a dosage of a fraction of the total nucleated cells required to be applied to the intended disease. There is no limitation on the number of cells stored in a unit so the units may contain equal or different dosages.

In another aspect, the characteristic may be determined by examining the proteome (the complete protein component of a cell). Proteome may be examined using conventional methods which include, at least, automated proteome analyzers or one, two, or multi dimensional gel electrophoresis. In another aspect, the characteristic may be determined by contacting the proteins of the cell or cell population with one or more antibodies. The antibody assay may be a sandwich assay, a strip (dipstick) assay, a western blot and the like.

The processing step may also involve treating the one population of stem cells or said at least one population of non-stem cells with an agent to enhance the storage, viability, or therapeutic ability of said cell population (i.e., a process of molecular reprogramming). The agent may be a nucleic acid, in which case treatment may involve transforming the cells with DNA or RNA. It is understood that transformation may be transient or permanent. Transient transformation involves introducing nucleic acids into a cell and allowing the cell to express the nucleic acid and make products such as RNA or proteins that may affect the function of the cells. It is understood that in a majority of cells, the transformed DNA is eventually lost and the effect of a transient transformation is thus of a limited duration. Permanent transformation is performed by using nucleic acids which would integrate into the genome of the host cell and become part of the genome in subsequent cellular division. The agent may also be a protein (ligand, antibody, and analogs and mimics thereof).

One advantage of the invention is that HLA typing is not necessary for the collected cells because the cells are used for autologous transfer. Since the transfer is autologous, graft vs. host or host vs. graft rejection is not a concern.

***Determining step***

In addition, the processing step may involve a step of determining from the collected population of cells at least a distinctive property associated with the person prior to storing in a the stem cell bank, so as to provide a means of secured identification to match the collected stem cells with the person at the time of use. The distinctive property may be a name, date of birth, social security number, and the like. In a more preferred embodiment, the distinctive property is a biological property such as a DNA or RNA sequence or a proteome of the person. It is understood that the DNA, RNA or proteome need not be complete. For example, a short stretch of a highly heterogeneous region of DNA is sufficient to identify a person. It is also understood that the distinctive property does not need to identify a person with 100% confidence. The distinctive property is used to assist in identification and while possible, does not have to be the sole means of identification. One distinctive property may be, for example, the presence of the Y chromosome.

In another embodiment, the determining step may further include providing an indicia with each unit of collected cell that represents the distinctive property. These indicia may be, for example, a label, bar code, magnetic strip, and microchip. These indicia may be embedded within a sample of preserved cells. For example, the indicia may be embedded in a bead mixed with the preserved cells. As another example, the indicia may be encoded into a nucleic acid, such as an oligonucleotide, that is mixed with the preserved cell. The oligonucleotide may comprise a known sequence allowing it to be easily amplified, sequenced, or both. Thus, the indicia may be identified, for example by taking a small sample of preserved cells for analysis. In the case of cryogenically preserved cells, a small sample may be scraped from the frozen cell population and analyzed without thawing the complete population of preserved cells.

*Stem Cell Enrichment or Sorting*

In one aspect of the invention, the cells collected by the methods of the invention may be sorted into at least two subpopulations which may be cryopreserved separately or together (*e.g*., in the same vial). The at least two subpopulations of cells may be two subpopulation of stem cells. However, the at least two subpopulation of cells may be (1) a stem cell population or a population enriched for stem cells and (2) a non stem cell population or a population depleted for stem cells. Furthermore, it is also envisioned that the two subpopulations (i.e., (1) and (2) above) may be cryopreserved together.

Stem cells may be sorted according to cell surface markers that are associated with stem cells. Since it is one embodiment of the invention to enrich for stem cells, useful markers for cell sorting need not be exclusively expressed in stem cells. A cell marker which is not exclusively expressed in stem cell will nevertheless have utility in enriching for stem cells. It should noted also that markers of differentiated cells are also useful in the methods of the invention because these markers may be used, for example, to selectively remove differentiated cells and thus enrich stem cells in the remaining cell population. Markers, cell surface or otherwise, which may be used in any of the processes of the invention include, at least, the following:

| **Marker** | **Cell Type** | **Significance** |
|---|---|---|
| | | **Blood Vessel** |
| Fetal liver kinase-1 (Flk1) | Endothelial | Cell-surface receptor protein that identifies endothelial cell progenitor; marker of cell-cell contacts |
| | | **Bone** |
| Bone-specific alkaline phosphatase (BAP) | Osteoblast | Enzyme expressed in osteoblast; activity indicates bone formation |
| | | **Bone Marrow and Blood** |
| Bone morphogenetic protein receptor (BMPR) | Mesenchymal stem and progenitor cells | Important for the differentiation of committed mesenchymal cell types from mesenchymal stem and progenitor cells; BMPR identifies early mesenchymal lineages (stem and progenitor cells) |
| CD34 | Hematopoietic stem cell (HSC), satellite, endothelial progenitor | Cell-surface protein on bone marrow cell, indicative of a HSC and endothelial progenitor; CD34 also identifies muscle satellite, a muscle stem cell |
| CD34⁺, Sca1⁺, Lin⁻ profile | Mesencyhmal stem cell (MSC) | Identifies MSCs, which can differentiate into adipocyte, osteocyte, chondrocyte, and myocyte |
| CD38 | Absent on HSC Present on WBC lineages | Cell-surface molecule that identifies WBC lineages. Selection of CD34+/CD38- cells allows for purification of HSC populations |
| c-Kit | HSC, MSC | Cell-surface receptor on BM cell types that identifies HSC and MSC; binding by fetal calf serum (FCS) enhances proliferation of ES cells, HSCs, MSCs, and hematopoietic |
| | | progenitor cells |
| Colony-forming unit (CFU) | HSC, MSC | Progenitor CFU assay detects the ability of a single stem cell or progenitor cell to give rise to one or more cell lineages, such as red blood cell (RBC) and/or white blood cell (WBC) lineages |
| Fibroblast colony-forming unit (CFU-F) | Bone marrow fibroblast | An individual bone marrow cell that has given rise to a colony of multipotent fibroblastic cells; such identified cells are precursors of differentiated mesenchymal lineages |
| Hoechst dye | Absent on HSC | Fluorescent dye that binds DNA; HSC extrudes the dye and stains lightly compared with other cell types |
| KDR | Hematopoietic stem cell and progenitor cell. | VEGF-receptor 2. Present in hematopoietic stem cells and progenitor cells. |
| Leukocyte common antigen (CD45) | WBC | Cell-surface protein on WBC progenitor |
| Lineage surface antigen (Lin) | HSC, MSC Differentiated RBC and WBC lineages | Thirteen to 14 different cell-surface proteins that are markers of mature blood cell lineages; detection of Lin-negative cells assists in the purification of HSC and hematopoietic progenitor populations |
| Muc-18 (CD146) | Bone marrow fibroblasts, endothelial | Cell-surface protein (immunoglobulin superfamily) found on bone marrow fibroblasts, which may be important in hematopoiesis; a subpopulation of Muc-18+ cells are mesenchymal precursors |
| Stem cell antigen (Sca-1) | HSC, MSC | Cell-surface protein on bone marrow (BM) cell, indicative of HSC and MSC Bone Marrow and Blood cont. |
| Stro-1 antigen | Stromal (mesenchymal) precursor cells, hematopoietic cells | Cell-surface glycoprotein on subsets of bone marrow stromal (mesenchymal) cells; selection of Stro-1+ cells assists in isolating mesenchymal precursor cells, which are multipotent cells that give rise to adipocytes, osteocytes, smooth myocyte, fibroblasts, chondrocytes, and blood cells |
| Thy-1 | HSC, MSC | Cell-surface protein; negative or low detection is suggestive of HSC |
| CD14 | monocytes | Monocyte differentiation to dendritic cells. |
| Platelet Endothelial Cell Adhesion Molecule (PECAM-1 or CD31) | neutrophils, macrophages | Endothelial cell adhesion |
| CD73 | Lymphocyte | Lymphocyte maturation cell marker |
| | | **Fat** |
| Adipocyte lipid-binding protein (ALBP) | Adipocyte | Lipid-binding protein located specifically in adipocyte |
| Fatty acid transporter (FAT) | Adipocyte | Transport molecule located specifically in adipocyte |
| Adipocyte lipid-binding protein (ALBP) | Adipocyte | Lipid-binding protein located specifically in adipocyte |
| | | **Liver** |
| B-1 integrin | Hepatocyte | Cell-adhesion molecule important in cell-cell interactions; marker expressed during development of liver |
| | | **Nervous System** |
| CD133 | Neural stem cell, HSC | Cell-surface protein that identifies neural stem cells, which give rise to neurons and glial cells |
| Glial fibrillary acidic protein (GFAP) | Astrocyte | Protein specifically produced by astrocyte |
| 04 | Oligodendrocyte | Cell-surface marker on immature, developing oligodendrocyte |
| CD166 | Neural cell marker | Neural cell marker; activated T-cells |
| | | **Pancreas** |
| Cytokeratin 19 | Pancreatic | CK19 identifies specific pancreatic epithelial cells that are progenitors for islet |
| (CK19) | epithelium | cells and ductal cells |
| Nestin | Pancreatic progenitor | Structural filament protein indicative of progenitor cell lines including pancreatic |
| | | **Pluripotent Stem Cells** |
| Alkaline phosphatase | Embryonic stem (ES) embryonal carcinoma (EC) | Elevated expression of this enzyme is associated with undifferentiated pluripotent stem cell (PSC) |
| Alpha-fetoprotein (AFP) | Endoderm | Protein expressed during development of primitive endoderm; reflects endodermal differentiation Pluripotent Stem Cells |
| Bone morphogenetic protein-4 | Mesoderm | Growth and differentiation factor expressed during early mesoderm formation and differentiation |
| Brachyury | Mesoderm | Transcription factor important in the earliest phases of mesoderm formation and differentiation; used as the earliest indicator of mesoderm formation |
| Cluster designation 30 (CD30) | ES, EC | Surface receptor molecule found specifically on PSC |
| Cripto (TDGF-1) | ES, cardiomyocyte | Gene for growth factor expressed by ES cells, primitive ectoderm, and developing cardiomyocyte |
| GATA-4 gene | Endoderm | Expression increases as ES differentiates into endoderm |
| GCTM-2 | ES, EC | Antibody to a specific extracellular-matrix molecule that is synthesized by undifferentiated PSCs |
| Genesis | ES, EC | Transcription factor uniquely expressed by ES cells either in or during the undifferentiated state of PSCs |
| Germ cell nuclear factor | ES, EC | Transcription factor expressed by PSCs |
| Hepatocyte nuclear factor-4 (HNF-4) | Endoderm | Transcription factor expressed early in endoderm formation |
| Nestin | Ectoderm, neural and pancreatic progenitor | Intermediate filaments within cells; characteristic of primitive neuroectoderm formation |
| Neuronal cell-adhesion molecule (N-CAM) | Ectoderm | Cell-surface molecule that promotes cell-cell interaction; indicates primitive neuroectoderm formation |
| Oct-4 | ES, EC | Transcription factor unique to PSCs; essential for establishment and maintenance of undifferentiated PSCs |
| Pax6 | Ectoderm | Transcription factor expressed as ES cell differentiates into neuroepithelium |
| Stage-specific embryonic antigen-3 (SSEA-3) | ES, EC | Glycoprotein specifically expressed in early embryonic development and by undifferentiated PSCs |
| Stage-specific embryonic antigen-4 (SSEA-4) | ES, EC | Glycoprotein specifically expressed in early embryonic development and by undifferentiated PSCs |
| Stem cell factor (SCF or c-Kit ligand) | ES, EC, HSC, MSC | Membrane protein that enhances proliferation of ES and EC cells, hematopoietic stem cell (HSCs), and mesenchymal stem cells (MSCs); binds the receptor c-Kit |
| Telomerase | ES, EC | An enzyme uniquely associated with immortal cell lines; useful for identifying undifferentiated PSCs |
| TRA-1-60 | ES, EC | Antibody to a specific extracellular matrix molecule is synthesized by undifferentiated PSCs |
| TRA-1-81 | ES, EC | Antibody to a specific extracellular matrix molecule normally synthesized by undifferentiated PSCs |
| Vimentin | Ectoderm, neural and pancreatic progenitor | Intermediate filaments within cells; characteristic of primitive neuroectoderm formation |
| | | **Skeletal Muscle/Cardiac/Smooth Muscle** |
| MyoD and Pax7 | Myoblast, myocyte | Transcription factors that direct differentiation of myoblasts into mature myocytes |
| Myogenin and MR4 | Skeletal myocyte | Secondary transcription factors required for differentiation of myoblasts from muscle stem cells |
| CD36 (FAT) | Cardiac cell marker | Integral membrane protein |
| | | **Progenitor Cell Marker** |
| CD29 | | Late antigen receptor involved in cell-cell adhesions |

The pattern of markers express by stem cells may also be used to sort and categorize stem cells with greater accuracy. Any means of characterizing, including the detection of markers or array of markers, may be used to characterized and/or identify the cells obtained through the embodiments disclosed herein. For example, certain cell types are known to express a certain pattern of markers, and the cells collected by the processes described herein may be sorted on the basis of these known patterns. The table that follows provides examples of the identifying pattern or array of markers that may be expressed by certain cell types.

| **Cell Type** | **Markers** |
|---|---|
| Hematopoietic stem cell | C34, CD45, CXCR4 |
| Endothelial Progenitors Cells | CD34, CD73, CD133, CXCR4, KDR, anti-M IgG |
| Very Small Embryonic Like Cell. (VSEL) | CD34, CD133, CXCR4, SSEA4, anti-M IgG |
| Mesenchymal Stem Cells | CD34, CD45, CD90, CD105, CD106, CD44 |

***In vitro Propagation of Stem Cells***

The peripheral blood stem cells of the present embodiments express markers of pluripotency. In response to appropriate differentiation signals, the stem cells differentiate along multiple pathways giving rise to many different phenotypes. According to another preferred embodiment, the stem cells may be induced to differentiate *in vitro* to cells that express at least one characteristic of a specialized tissue cell lineage. The non-neonatal or adult stem cells of the present embodiments may be induced to partially or totally differentiate into tissue cells having the features of tissue cells that include, but not limited to, endocrine pancreas, exocrine pancreas, brain, liver, cartilage, bone, muscle, heart, and kidney.

The stem cells collected from the apheresis process or enriched populations of cells may be propagated or differentiated *in vitro.* The stem cells may be differentiated by placing the cells under the influence of signals designed to induce specifically the foregoing phenotypes. Any method of subjecting the stem cells to such signals may include, but not limited to, transfection of stem cells with genes known to cause differentiation, and/or exposing the stem cells to differentiation agents. For example, the stem cells may be genetically modified either stably or transitorily to express exogenous genes or to repress the expression of endogenous genes. In such a manner, the differentiation of the stem cells may be controlled. As an alternative example, the stem cells, and colonies thereof, may be induced to differentiate along a predictable pathway through the use of media that favors the maintenance in culture of a phenotype.

The propagation of the stem cells may be achieved using any known method. The stem cells of the present invention may be propagated on: 1) a tissue culture substrate in a stem cell medium that favors the maintenance of stem cells in a undifferentiated or dedifferentiated condition; 2) on fibroblast feeder layers that support cell growth and proliferation and inhibition of differentiation; or 3) a combination of both 1 and 2. In a preferred embodiment, the tissue culture substrate is coated with an adhesive or other compound or substance that enhances cell adhesion the substrate (e.g., collagen, gelatin, or poly-lysine, etc.). Collagen-coated plates are most preferred. Where fibroblast feeder cells are utilized, mouse or human fibroblasts are preferably used; alone or in combination. It is preferred that the feeder cells are treated to arrest their growth, which may be accomplished by irradiation or by treatment with chemicals such as mitomycin C that arrests their growth. Most preferably, the fibroblast feeder cells are treated with mitomycin C. In preferred embodiments, the fibroblast feeder layer has a density of approximately 25,000 human and 70,000 mouse cells per cm², or 75,000 to 100,000 mouse cells per cm². Preferably, the stem cells are cultured for a period of 4 to 24 days, and preferably for a period of 7 to 14 days. Preferably, the stem cells are grown on a fibroblast feeder layer, such as mitomycin treated MEF cells, for a period of about 4 to 14 days, and preferably from 7 to 10 days.

***Cellular Therapy***

In one embodiment of the present invention, the stem cells are collected from the peripheral blood of a subject and introduced or transplanted back to the individual when the subject is in need of such cellular therapy. The stem cells of the present invention may further be isolated and enriched to contain a large number of stem cells showing long-term survival following transplantation.

Stem cells and compositions comprising stem cells of the present invention can be used to repair, treat, or ameliorate various aesthetic or functional conditions (e.g. defects) through the augmentation of damage tissues. The stem cells of the present embodiments may provide an important resource for rebuilding or augmenting damaged tissues, and thus represent a new source of medically useful stem cells. In a preferred embodiment, the stem cells may be used in tissue engineering and regenerative medicine for the replacement of body parts that have been damaged by developmental defects, injury, disease, or the wear and tear of aging. The stem cells provide a unique system in which the cells can be differentiated to give rise to specific lineages of the same individual or genotypes. The stem cells therefore provide significant advantages for individualized stem cell therapy.

In addition, such stem cells and compositions thereof can be used for augmenting soft tissue not associated with injury by adding bulk to a soft tissue area, opening, depression, or void in the absence of disease or trauma, such as for "smoothing". Multiple and successive administrations of stem cells are also embraced by the present invention.

For stem cell-based treatments, a stem cells are preferably collected from an autologous or heterologous human or animal source. An autologous animal or human source is more preferred. Stem cell compositions are then prepared and isolated as described herein. To introduce or transplant the stem cells and/or compositions comprising the stem cells according to the present invention into a human or animal recipient, a suspension of mononucleated cells is prepared. Such suspensions contain concentrations of the stem cells of the invention in a physiologically-acceptable carrier, excipient, or diluent. For example, suspensions of stem cells for administering to a subject preferably comprise 10⁸ to 10⁹ cells/ml in a sterile solution of complete medium modified to contain the subject's serum, as an alternative to fetal bovine serum. Alternatively, stem cell suspensions may be in serum-free, sterile solutions, such as cryopreservation solutions. Enriched stem cell preparations may also be used. The stems suspensions may then be introduced e.g., via injection, into one or more sites of the donor tissue.

Concentrated or enriched cells may be administered as a pharmaceutically or physiologically acceptable preparation or composition containing a physiologically acceptable carrier, excipient, or diluent, and administered to the tissues of the recipient organism of interest, including humans and non-human animals. The stem cell-containing composition may be prepared by resuspending the cells in a suitable liquid or solution such as sterile physiological saline or other physiologically acceptable injectable aqueous liquids. The amounts of the components to be used in such compositions can be routinely determined by those having skill in the art.

The stem cells or compositions thereof may be administered by placement of the stem cell suspensions onto absorbent or adherent material, *i.e.*, a collagen sponge matrix, and insertion of the stem cell-containing material into or onto the site of interest. Alternatively, the stem cells may be administered by parenteral routes of. injection, including subcutaneous, intravenous, intramuscular, and intrasternal. Other modes of administration include, but are not limited to, intranasal, intrathecal, intracutaneous, percutaneous, enteral, and sublingual. In one embodiment of the present invention, administration of the stem cells may be mediated by endoscopic surgery.

For injectable administration, the composition is in sterile solution or suspension or may be resuspended in pharmaceutically- and physiologically-acceptable aqueous or oleaginous vehicles, which may contain preservatives, stabilizers, and material for rendering the solution or suspension isotonic with body fluids (i.e. blood) of the recipient. Non-limiting examples of excipients suitable for use include water, phosphate buffered saline, pH 7.4, 0.15 M aqueous sodium chloride solution, dextrose, glycerol, dilute ethanol, and the like, and mixtures thereof. Illustrative stabilizers are polyethylene glycol, proteins, saccharides, amino acids, inorganic acids, and organic acids, which may be used either on their own or as admixtures. The amounts or quantities, as well as the routes of administration used, are determined on an individual basis, and correspond to the amounts used in similar types of applications or indications known to those of skill in the art.

Consistent with the present invention, the stem cells may be administered to body tissues, including epithelial tissue (i.e., skin, lumen, etc.) muscle tissue (i.e. smooth muscle), blood, brain, and various organ tissues such as those organs that are associated with the urological system (i.e., bladder, urethra, ureter, kidneys, etc.).

According to another preferred embodiment, there is provided compositions and methods for enhancing engraftment of the peripheral blood stem cells. The cells collected from the peripheral blood of a subject may generally comprise a comprehensive mixture of cells. That is, there exist a mixture of stem cells, partially differentiated cells (*e.g.*, progenitor cells or fibroblasts), and functional cells (*i.e.*, terminally differentiated cells). The presence of progenitor cells, partially and possibly, terminally differentiated cells may have significant advantages with respect to a shorter time to reconstitution and other physiological benefits in the post-infusion period.

According to the general treatment method described herein, the cellular mixture, obtained through an apheresis process, may be administered to a subject, for example, by infusion into the blood stream of a subject through an intravenous (i.v.) catheter, like any other i.v. fluid. Alternatively, however, an individualized mixture of cells may be generated such as to provide a cellular therapy mixture specific for therapeutic needs of a subject. The comprehensive mixture of cells obtained such as through an apheresis process may be characterized, sorted, and segregated into distinct cell populations. Cell markers such as stem cells markers or tissue specific markers may be used to phenotypically characterize the populations of cells collected from the peripheral blood. Using these markers, it is possible to segregate and sort on the basis of cell type. The mixture of cells is thus transformed into populations of cells, which may be broadly classified into two portions: a stem cell portion and a non-stem cell portion. The non-stem cell portion may further be classified into a progenitor cell or fibroblast portion and a function cell or fully differentiated cell portion. Once the peripheral blood cellular mixture is sorted, the stem cell and non-stem cell portions may be cryopreserved and stored separately. In this manner, a library or repository of distinct cell populations from a subject may be created. Alternatively, stem cell and non-stem cell portions may the cryopreserved together and then sorted and separated prior to use.

The types of cell populations that may be generated in this manner include any population of a cell type that developed from a germ layer (*i.e*., endoderm, mesoderm, and ectoderm). These include, but are not limited to, peripheral blood stem cells, hematopoietic progenitor or differentiated cells, neural progenitor or differentiated cells, glial progenitor or differentiated cells, oligodendrocyte progenitor or differentiated cells, skin progenitor or differentiated cells, hepatic progenitor or differentiated cells, muscle progenitor or differentiated cells, bone progenitor or differentiated cells, mesenchymal stem or progenitor cells, pancreatic progenitor or differentiated cells, progenitor or differentiated chondrocytes, stromal progenitor or differentiated cells, cultured expanded stem or progenitor cells, cultured differentiated stem or progenitor cells, or combinations thereof. Of particular interest are hematopoietic cells, which may include any of the nucleated cells which may be involved with the erythroid, lymphoid or myelomonocytic lineages, as well as myoblasts and fibroblasts. Also of interest are progenitor cells, such as hematopoietic, neural, stromal, muscle (including smooth muscle), hepatic, pulmonary, gastrointestinal, and mesenchymal progenitor cells. Also of interest are differentiated cells, such as, osteoblasts, hepatocytes, granulocytes, chondrocytes, myocytes, adipocytes, neuronal cells, pancreatic, or combinations and mixtures thereof.

The collected stem cells and/or progenitor cells also may be expanded using an *ex-vivo* process. For example, it may be necessary to expand and propagate a population of stem cells, partially differentiate stem cells to achieve a population of tissue specific progenitor cells, or to differentiate stem cells or progenitor cells into fully functional cells. A variety of protocols have been developed for the enrichment of such populations. See *e.g.*, U.S. Patent No. 5,486,359, U.S. Patent No. 5,753,506, and U.S. Patent No. 5,736,396, incorporated herein by reference in their entireties. Any known protocol for the expansion or differentiation of stems cells or progenitor cells may be employed. For example, strategies employed may include culturing stem cells or progenitor cells: with or without different cocktails of early and late growth factors; with or without tissue specific growth or differentiation factors; with or without serum; in stationary cultures, rapid medium exchanged cultures or under continuous perfusion (bioreactors); and with or without an established cell feeder layer. In order to achieve maximal *ex-vivo* expansion of stem cells the following general conditions should be fulfilled: (i) differentiation should be reversibly inhibited or delayed and (ii) self-renewal should be maximally prolonged. Similarly, following cell expansion, it is important to have methods to induce differentiation of the expanded cell population, so as to covert the expanded cell population to mature functional cells or tissue.

The cell populations of the various cells types may then be combined, recombined, or compounded into a cellular therapy mixture of cells appropriate for treating the disease of a subject and/or regenerating a specific tissue. A combination of stem cells, tissue specific progenitor cells, and optionally functional cells is thought to enhance the engraftment of the stem cells. Accordingly, in one embodiment, the present invention provides methods and products for using an autologous mixture of stem cells, progenitor cells, and optionally functional cells to enhance engraftment of stem or progenitor cells. This cellular therapy product may comprise: from about 10% to about 90% peripheral blood stem cells, about 10% to about 80% peripheral blood stem cells, about 10% to about 60% peripheral blood stem cells, or about 10% to about 40% peripheral blood stem cells; and from about 10% to about 90% non-stem cells, from about 20% to about 90% non-stem cells, from about 40% to about 90% non-stem cells, from about 60% to about 90% non-stem cells. The non-stem portion may optionally comprise from about 5% to about 50% functional cells, about 5% to about 40% functional cells, about 5% to about 30% functional cells, about 5% to about 20% functional cells, or about 5% to about 10% functional cells.

A suitable example of the cellular therapy product described above is the autologous mixture of PBSCs, hematopoietic progenitor cells, and optionally granulocytes or other functional cell of the hematopoietic system. Another example is a cellular therapy product comprising an autologous mixture of PBSCs, myocardial progenitor cells, and optionally myocardial cells.

According to another preferred embodiment, there is provided a method of treating a patient in need thereof comprising administering to a subject an autologous mixture of stem cells, progenitor cells, and optionally functional cells. For example, the present invention is useful to enhance the effectiveness of hematopoietic progenitor cell engraftment as a treatment for cancer. The treatment of cancer by x-irradiation or alkylating therapy destroys the bone marrow microenvironment as well as the hematopoietic stem cells. The current treatment is to transplant the patient after marrow ablation with hematopoietic progenitor cells that have been previously harvested and cryopreserved. Because the bone marrow microenvironment is destroyed, however, hematopoietic progenitor cell engraftment is delayed until the stromal environment is restored. The compositions and methods discloses herein are useful in restoring the stromal environment and thereby enhancing the engraftment process.

***Stem Cell Banking***

In another aspect of the present invention, the current invention provides a cell bank to support an elective healthcare insurance model to effectively protect members of the population from future diseases. An individual can elect to have his or her own stem cells collected, processed and preserved, while he or she is in healthy state, for future distribution for his or her healthcare needs.

Collected and processed stem cells are "banked" for future use, at a stem cell bank or depository or storage facility, or any place where stem cells are kept for safekeeping. The storage facility may be designed in such a way that the stem cells are kept safe in the event of a catastrophic event such as a nuclear attack. In some embodiments, the storage facility might be underground, in caves or in silos. In other embodiments, it may be on the side of a mountain or in outer space. The storage facility may be encased in a shielding material such as lead.

According to a preferred embodiment, there is provided a process of stem cell banking with four steps. Step A involves administrating one or more stem cell potentiating agents to a person to increase the amount of stem cells in the peripheral blood of the person. Step B involves collecting at least one population of stem cells and at least one population of non-stem cells from peripheral blood of said person using an apheresis process, wherein said person has no immediate perceived health condition requiring treatment using his own collected stem cells. Step C involves preserving the at least one population of stem cells and the at least one population of non-stem cells as a preserved populations of cells. Step D involves retrieving the preserved populations of cells for autologous transplantation of the stem cells into the person. Each aspect of this process is described in more detail below.

**a. Unitized Storage**

The physical steps of stem cell storage, including use of cryo-protectant (DMSO), controlled rate freezing and storage within a liquid nitrogen filled tank may comprise the prior art. The inventive aspect of the stem cell storage process is directed to the concept of unitized storage permits the storage of stem cells in multiple locations, either above or below ground. Such locations can be selected such that they are secure from physical events such as fires or earthquakes or other act of nature and from terrorist attack or acts of war. In addition, unitized storage facilitates the removal and use of only the necessary number of stem cell units for treatment, thus leaving other units for future use.

Specifically, unitized storage involves the banking of the harvested stem cells in separate storage containers (bags, tubes, etc) of desired, defined units or dosages. At the time of use, only the required dosage is retrieved, by selecting the number of containers necessary to fulfill the desired dosage. Certain diseases may require stem cell therapy that includes a series of repeated treatments. By providing unitized storage of harvested stem cells, only the required dosage is retrieved for each treatment, to complete the entire therapy.

The number of units of stem cells for each storage container can be predetermined, in accordance with general prevailing stem cell therapy and treatment requirements, or in accordance with consideration of specific diseases anticipated to require stem cell therapy. For example, depending on the health condition, genetic history and/or profile of the donor, certain specific diseases may be targeted to potentially require or benefit from stem cell therapy in the future. Depending on the particular diseases targeted, the units required for each stem cell therapy treatment can be estimated before hand, so that each separate storage container is filled with no more than the more likely amount to be used in the future. Each container does not necessarily contain the total amount expected to be used in a future treatment. The total amount of collected stem cells may be subdivided into defined fractional units in smaller containers, such that several containers of stem cells may be used to make up the total needed for a particular treatment.

Unitized storage for multiple dosage concept of the present invention is made possible only by the present invention, in that the inventive concept of elective collection and banking of autologous peripheral blood stem cells during pre-disease stage enables sufficient quantity and quality of harvested stem cells to be unitized into separate storage containers, each containing a prescribed number of units of stem cells. Generally, it may be desirable to bank at least 20 containers or units of stem cell for future stem cell therapy to treat certain diseases. Prior art allogeneic stem cell collection (*e.g*., from umbilical cords) simply does not result in sufficient quantity of stem cells, and certainly not in such quantity, and further not in a quality that would be effective.

Another inventive aspect of the invention is that each of the storage containers (*e.g*., bags or tubes) will be tagged with positive identification based on a distinctive property associated with the subject prior to storing in a stem cell bank. For example, DNA genetic fingerprint and HLA typing may be used with secured identification mechanism such as acceptable methods using microchips, magnetic strip, and/or bar code labels. This identification step 40 may be included in the process 30 in Figure 3. Prior to use of the stem cells, a DNA sample is taken from the patient and compared to the DNA genetic fingerprint identification on the bags. This approach provides positive identification of the correct banked stem cells that originated from the particular patient.

One advantage of the method is that the collected cells are preserved before substantial cell division *in vitro.* That is, the preserved cells are mostly or completely comprised of "primary cells." Primary cells are defined as cells that have not undergone cellular division *in vitro.* The preservation may involve preserving cells in multiple separate containers for storage. The multiple containers may contain any combination of cells. For example, each container may contain cells collected from one apheresis session. Alternatively, each container may contain a population of stem cells, a population of non-stem cells, a population enriched for stem cells, or a population enriched for non stem cells. Each container may also contain a cell population enriched or depleted for a cell with a specific cell surface antigen. The cell surface antigens that can be enriched or depleted include any antigen of this disclosure including, at least, CD34, CD 133, and KDR. In a preferred embodiment, the cell surface antigen is an antigen that can distinguish between a stem cell and a non-stem cell. This could be, for example, an antigen that is specific for a non-stem cell. Stem cells may be enriched by excluding cells with this cell surface antigen. Alternatively, the antigen may be stem cell specific and stem cells may be selected based on the presence of the antigen. In another embodiment, the antigen may be a progenitor cell surface antigen, or an antigen associated with terminally differentiated cells.

A preferred method of cell preservation is cryogenic storage which can involve storage of cells at liquid nitrogen temperatures at about -196 degrees centigrade to about -80 degrees centigrade. Methods for the cryopreservation of cells are known in the art.

The preserving step may comprise storing the collected stem cells in a stem cell bank. For example, the preserving stem may comprise the step of processing the stem cells, including unitizing the collected stem cells into multiple separate units of containers. The preserving step may be performed independent of tissue or HLA typing of the collected stem cells prior to storing in the stem cell bank. Further, the preserving step may comprise the step of determining from the collected stem cells at least a distinctive property associated with the person prior to storing in a the stem cell bank, so as to provide a means of secured identification to match the collected stem cells with the person at the time of use. HLA typing step may include providing an indicia with each unit representing information of said at least one distinctive property. For example, the indicia may be embodied in at least one of a label, bar code, magnetic strip, microchip and a nucleic acid based sequence (discussed in more detail below).

***Transplantation-Treatment Using Banked Stem Cells***

The health conditions that can be treated by the methods of the invention include any disease where stem cells are used for treatment. Preferably, the disease is a cancer or other disease where treatment is benefited by the restoration of hematopoietic cells levels in a subject. For example, the treatment of many cancers (neoplastic disorders) requires chemotherapy followed by reconstitution of one or more cell systems in the body using stem cells. The most common system that requires reconstitution following chemotherapy is the hematopoietic system. Other diseases that can benefit from stem cell treatment include immune diseases (including cancer and autoimmune diseases), and leukopenia.

As stated above, the reserved cells of the invention may be transferred to a person as needed in an autologous transfer. The transfer may be used, for example, to treat a person in a leukopenic state. The methods of the invention may be used to treat persons in acute need of immune system augmentation. This may arise, for example, in a person with toxic shock from bacterial infections.

According to a preferred embodiment, there is provided a process for treatment of a person, comprising the steps of the person proactively electing to have his stem cells collected with no immediate perceived health condition requiring treatment using his own stem cell; collecting stem cells from the person; at the time of collection, earmarking the collected stem cells for use by the person; preserving the collected stem cells in storage; retrieving the stored stem cells if and when the person is diagnosed with a disease requiring stem cell treatment; and treating the person using his own stem cells retrieved from storage. In this process, the electing step may include considering a targeted disease or a class of diseases to which the collected stem cells are intended to be applied for treatment, and wherein at the time of collection, the person is not diagnosed with such targeted disease or diseases. The collecting step may include collecting stem cells in such quantity as to be sufficient in anticipation of the treatment. Furthermore, this process may further comprise the steps of: determining from the collected stem cells at least a distinctive property associated with the person prior to storing in a the stem cell bank, so as to provide a means of secured identification to match the collected stem cells with the person at the time of use; and at the time of use of the stored stem cells, matching the distinctive property with a sample from the person to positively identify the stored stem cells as being collected from the person.

In another embodiment, there is provided a process for treatment of a person, comprising the steps of: the person proactively electing to have his stem cells collected with no immediate perceived health condition requiring treatment using his own stem cell; collecting stem cells from the person; applying the collected stem cells after the person has been diagnosed with a disease requiring stem cell treatment; and treating the person using his own collected stem cells.

Banked stem cells may be applied to treatment of a patient who was the subject of the stem cell collection process. Conventional standard transfusion methods (e.g. intravenous infusion) may be used for infusing the stem cells to a patient. Standard protocols for chemotherapy may be used followed by stem cells infusion for bone marrow reconstitution.

According to the present invention, the distribution of delivery of stem cells into a patient may be accomplished by any one of the conventional known infusion processes.

Normal conventional practice should be observed to monitor the progress of the patient undergoing transplantation. However, the applicants' method should reduce the amount ofpotential complications resulting from immune rejection, graft-versus-host-disease, the duration of engraftment and infectious complications. The patient would benefit significantly because, if engraftment and reconstitution of the hematopoietic system does not occur after transplantation, the physician can rapidly detect this rejection and proceed with a second transplant.

There are many potential uses for the cells and methods of the invention. For example, it is possible to generate healthy heart muscle cells in the laboratory and then transplant those cells into patients with chronic heart disease. Alternatively, it is possible to inject stem cells directly into a patient to generate new heart muscle cells. Research in mice and clinical trials in human and other animals indicates that bone marrow stem cells, transplanted into a damaged heart, can generate heart muscle cells and successfully repopulate the heart tissue. Other recent studies in cell culture systems indicate that it may be possible to direct the differentiation of embryonic stem cells or adult stem cells into heart muscle cells.

In people who suffer from type I diabetes, the cells of the pancreas that normally produce insulin are destroyed by the patient's own immune system.

Study have shown that it may be possible to direct the differentiation of human stem cells in cell culture to form insulin-producing cells that eventually could be used in transplantation therapy for diabetics.

In another embodiment, autologous adult or non-neonate child peripheral blood stem cells at doses below those used in the therapy of the above diseases and disorders, can be used without ablation to serve as boosters for the immune system in individuals to whom the immune system, is depressed due to illness, infections, stress, aging or other factors.

In the process, the electing step may include the step of considering a targeted disease or a class of diseases to which the collected stem cells are intended to be applied for treatment. The collecting step may be undertaken at a time when the person is in a pre-disease stage, including at least the stage in which the person has not been diagnosed of the targeted disease or diseases to which the collected stem cells are intended to be applied for treatment. Further, the collecting step may collect stem cells in sufficient quantity that is anticipated to be needed for such treatment. In a preferred embodiment, the collecting step is conducted when (1) the person is an adult or a non-neonate child and/or (2) meets at least one of a prescribed weight and age, and/or (3) meets at least one of the following conditions: between 10-200 Kg weight and between 2 to 80 years old. In another preferred embodiment, the collecting step is undertaken over multiple sessions, at least one of different ages and weights of the person. In another embodiment, the collecting step includes the step of collecting at least on the order of 10⁶ total nucleated cells per kilogram weight of the person in a single collection session.

Thus, in one embodiment of the invention: the stem cells of a non-neonate child or an adult ("person"), while the non-neonate child or adult is in a pre-disease state, are harvested and then preserved (such as cryopreservation). The harvesting (collection) process can be achieved using apheresis. There may be a need to infuse cell growth factors, such as G-CSF, 1-6 days prior to the collection. Accordingly to a preferred embodiment, the cell growth factors are administered to the non-neonate child or adult on two consecutive days followed by the collection of the peripheral blood stem cells on day 3 by an apheresis process. To preserve the stem cells collected for future used, cryopreservation technique and reagent can be used.

Later (*e.g*., years later), should the same person develops cancer, an immunodisease, infectious disease, heart disease, brain disease, spiral cord disease, pancreatic disease, hepatic disease or bone marrow disease or undergoes therapy or is exposed to conditions which causes immunosuppression or infection or depletion of his immune cells, then the preserved stem cells or bone marrow are infused into the person to combat the disease. This may be achieved by intravenous infusion, intra arterial, intra-organ injection, intra bone marrow injection, intra-fat injection, intra-muscle injection of the stem cell products, or by intramedulary infusion (bone marrow), selective arterial infusion, pericardial infusion, epidural and subdural infusions. Similarly, the treatment protocol, and the criteria for determining the progress of the person and for adjusting the amount/dosage of cells to be infused may be achieved using standard transplantation practice.

Of course, the amount of stem cells collected should be sufficient for a major transplantation. If necessary, multiple collections should be done at an appropriate interval between collections (may be one week or more apart). However, as medicine advances, the preserved cells can be ex-vivo expanded and made to multiply or differentiate into the desired cell types before infusion into the person. If the person is deficient in certain subpopulation of cells, the subpopulation of cells from the preserved or expanded cells may be selected for in the future, and infused into the person. Furthermore, the harvested or expanded cells may be programmed by growing them *in vitro* with the person's diseased cells or tissues, or under stimulation by desired chemicals or cytokines before selecting for the desirable programmed cell and infusing them into the person.

In this embodiment, stem cells and bone marrow cells are chosen because they are versatile and because of their known use in cancer and immunodisease treatments and known methods for harvesting, processing, preserving, expanding them. Their use in such treatments may be employed in this invention. The following describes this embodiment in further details.

By way of example, and not limitation, an application of banked stem cells is described in reference to cancer treatment.

**a. Development of Stem Cell Treatment of Cancer**

Mechanisms that cause normal tissues to become malignant involve an "enormously complex process". Indeed, complexity in carcinogenesis occurs at each of many hierarchical levels. Even at the genetic level, tumor cells accumulate mutations in multiple genes during formation of most cancer types. Cancer is also the outcome of altered mechanisms occurring at other levels involving RNA, proteins, intracellular pathways, intercellular interactions, tissues, organs, etc. Since events occurring at one hierarchical level feed into and modify mechanisms at other levels, cancer development is a dynamic process that is more complicated than a simple summation of the parts. An important consideration is that some cancers may originate from or associated with stem cells.

Thus, for cancer patients who face immunosuppressive therapy who have no readily matched donor, doctors have used "autologous" transplants: the cancer patient's bone marrow is removed, frozen, and stored prior to chemotherapy and/or radiation. Then the cells are thawed and reinfused into the patient after chemotherapy and/or radiation.

The collection of stem cell products (SC products), a term which includes both true stem cells and committed progenitor cells (i.e., CD 34+ cells are included), whether from bone marrow, cord blood or peripheral blood from third party donors, can be stored for future use, one of the most significant uses of stem cells is transplantation to enhance hematological recovery following an immunosuppressive procedure such as chemotherapy.

In the prior art, there is one significant drawback to the use of this very beneficial reinfusion procedure for treating a cancer patient. When SC products are obtained from the cancer patient, a significant number of tumor cells may also be collected, thereby contaminating the SC product.

Subsequently, when the SC product is reinfused into the cancer patient, the tumor cells are also reintroduced, increasing or re-introducing tumor cells into the patient's blood stream. While circulating tumor cells have not been directly linked to the relapse of a particular cancer, in the case of lymphoma, for example, reinfused cells have been traced to sites of disease relapse. In cases involving adenocarcinoma, it has been estimated that for a 50 kilogram adult, approximately 150,000 tumor cells can be reinfused during a single stem cell transplantation. Moreover, it has been shown that the tumor cells present in the SC product are viable and capable of *in vitro* clonogenic growth, thus suggesting that they could indeed contribute to post-reinfusion relapse. Ovarian cancer cells, testicular cancer cells, breast cancer cells, multiple myeloma cells, non-Hodgkin's lymphoma cells, chronic myelogenous leukemia cells, chronic lymphocytic leukemia cells, acute myeloid leukemia cells, and acute lymphocytic leukemia cells are known to be transplantable.

The extent of tumor cell contamination of SC products appears to vary greatly from patient to patient, and values within the range of 11 to 78 percent have been recorded. Therefore, the reinfusion of circulating tumor cells may well circumvent the benefits provided by aggressive chemotherapy followed by stem cell transplantation.

Methods currently used to separate the valuable stem cells from the undesired tumor cell-contaminated product rely on positive or negative selection techniques. Positive selection assays identify stem cells and progenitor cells that express markers for the CD34 antigen and remove them from the blood or bone marrow product contaminated with tumor cells. These methods are very labor intensive, reduce the number of useable stem cells and require the use of specialized equipment, thus greatly increasing the cost of patient care and severely limiting the use of SC products in transplantation procedures. An alternative to positive selection for removal of tumor cells from blood was provided by Gudemann *et al.,* who described filtration with special leukocyte depletion membrane filters (which work by adsorbing charged particles) to remove urologic tumor cells from autologous blood during an intraoperative mechanical autotransfusion (IAT) procedure. Gudemann *et al.,* Intraoperative Autotransfusion In Urologic Cancer Surgery By Using Membrane Filters, XXIII^{rd} Congress of the ISBT, abstracts in Vox Sang., 67 (S2), 22.), incorporated herein by reference in its entirety. A disadvantage of the membrane filters used by Gudemann et al is that they do not selectively retain tumor cells. White blood cells, including stem cells, are also retained. Thus, tumor cells are not removed from stem cells. The work of Miller *et al.* also teaches that standard blood transfusion filters are ineffective at removing tumor cells from autologous blood. Miller et al., Autologous transfusion: an alternative to transfusion with banked blood during surgery for cancer, B. J. Surg. 1991, Vol. 78, June, 713-715, incorporated herein by reference in its entirety.

On another front, in an attempt to improve the efficacy of stem cell therapy, scientists have exposed the cancer patients' extracted stem cells to modification in culture medium in the hope of "programming" them, such as to enhance their cancer fighting capability, before transfusing them into the patient. However, such studies have been unsuccessful in demonstrating a superiority of programmed stem cells versus native stem cells clinically.

**b. Development of a Solution in Accordance with the Invention**

Applicants see a need to improve the benefits of stem cell transfusion, which would ultimately result in increased survival rates, while at the same time providing a low-cost, clinically effective method for treating cancer patients with stem cell products. Prompted by such, applicants created the inventive methods disclosed herein based on certain initial hypotheses, which hypotheses may or may not be relevant to various embodiments of the inventive methods ultimately developed. Without wishing to be bound by the hypotheses postulated in this application, applicants made the following hypotheses. Each hypothesis may or may not relate to the other hypotheses. The efficacy of the invention in practice is obviously not bound by the correctness of the hypotheses.

As a first hypothesis, applicants believe that many cancers are systemic in nature at the time of diagnosis and widely distributed throughout the body. That is, applicants believe that by the time a patient has been diagnosed with many types of cancer, *e.g.*, breast cancer, there can already exist cancer cells in other parts of the patient besides the perceived affected area. In fact, the cancer may have already spread or migrated throughout the patient's body, for example, as the cancer cells are being carried by the patient's circulating blood or lymphoid system. This hypothesis accounts for contamination of stem cells, by cancer cells, collected from the patient, which may cause relapse after transplantation.

As a second hypothesis, applicants believe that in some instances, malignant or pre-malignant cells are routinely generated by the human body. However, the human does not develop cancer because his normal cells "self-regulate" the body by monitoring and eliminating the malignant or pre-malignant cells before they proliferate uncontrollably and give rise to cancer. This is termed "immune surveillance". Applicants further postulate that in some cancer patients, their previously healthy cells become diseased because their diseased cells have partially or completely lost the ability to "self-regulate" due to old age, and/or environmental assaults (exposure to radiation, carcinogens, or stress, etc.); and/or other factors as yet unknown. Thus, transplantation of such already diseased (defective) cells harvested from these patients may not be helpful.

As a third hypothesis, applicants believe that certain diseases arise due to the loss of one or more functions of a healthy cells, due to old age, and/or environmental assaults (exposure to radiation, carcinogens, or stress, etc.); and/or other factors as yet unknown. Such loss may result in the failure to self-regulate, or to generally sustain normal functioning of the body. For example, the cell loses the ability to produce an enzyme or chemical necessary for the body's proper functioning. Thus, such a loss may result in Alzheimer's disease, Parkinson's disease, etc.

In summary, based on the above hypotheses, even though the present application uses cancer as an example of a disease for treatment under the invention, it is understood that other diseases (which result from the partial or complete loss of one or more abilities of a cell over time; or a systemic disease; or immunodiseases, such as cancer) will similarly benefit from the present invention. Cancer is used herein merely for the convenience of illustration and discussion. The methods of the present invention can also be used to supplant immune cells to patients undergoing immunosuppressive treatments, such as chemotherapy, radiation therapy, or those who have been exposed to factors, which deplete their bodies of immune cells.

As a fourth hypothesis, the applicants believe that the programmed stem cells collected from cancer patients have not shown any observable advantage over unprogrammed stem cells from the same patient, because both the programmed and unprogrammed cells are already diseased and thus damaged. That is, both the programmed and unprogrammed cells have lost their cancer fighting ability (or their optimal cancer fighting ability as compared to healthy cells), and the "programming" cannot restore the normal function to the already diseased cells (which have irretrievably lost their function) necessary to fight cancer. Again, this hypothesis can be generalized to any disease, besides cancer, wherein a healthy cell will be more readily programmed that a diseased cell (which may be partially or completely unresponsive to programming).

**c. Cancer Treatment**

For ease of discussion, the following use breast cancer as a non-limiting example of cancer. The incident of breast cancer is the second highest, after lung cancer, in Caucasian women. Breast cancer is a difficult disease to treat. Patients undergoing chemotherapy, radiotherapy, or immunosuppressive therapy, generally lose immune cells. In the present invention, the patient's immune cells are replenished by his previously harvested pre-disease SC. Further, chemotherapy and radiotherapy destroy rapidly dividing cells which include cells found in bone marrow, the gastrointestinal tract (GI), and hair follicles. Thus, there is a threshold to the amount of chemical or radiation administered to the patient. Thus, with the stem cells replacement of this invention, a higher and more effective (aggressive) dose of chemotherapy or radiation may be administered to the patient to more aggressively eliminate the cancer cells.

(i) Methods For SC Collection, Processing, Preservation and Infusion

Conventional methods for collecting, processing, cryopreserving, storing thawing, screening for and quantifying stem cells, and selecting for subpopulations of the stem cells, may be used.

Apheresis collection process of peripheral blood stem cells is a common method for collecting stem cells today. Hematopoietic cells can be isolated from human tissues including, for example, peripheral blood, bone marrow, fat tissue. Mononuclear cells, for example peripheral blood mononuclear cells (PBMCs) may be further isolated by methods such as density-gradient centrifugation. Sufficient quantity should be collected. If necessary, multiple collections should be considered to ensure enough dose for most demanding transplantation, typically about one (1) billion cells. The stem cells may be preserved by cryopreservation and later thawed for use, using standard transfusion procedures.

In an embodiment of the invention, all the stem cells collected can be cryogenically preserved, and used for hematopoietic reconstitution after thawing, in order to avoid cell losses associated with cell separation procedures. However, it is envisioned that cell separation procedures can be used if desired.

In one embodiment of the present invention for the primitive cell population to be further subdivided into isolated subpopulations of cells that are characterized by specific cell surface markers. The methods of the present invention may further include the separation of cell subpopulations by methods such as high-speed cell sorting, typically coupled with flow cytometry.

(ii) Infusion and Transplantation

Conventional standard transfusion methods (*e.g*. intravenous infusion) may be used for infusing the stem cells. Standard protocols for chemotherapy may be used followed by stem cells infusion for bone marrow reconstitution.

(iii) Confirmation that the Transplant is Working

Normal conventional practice should be observed to monitor the progress of the patient undergoing transplantation. However, the applicants' method should reduce the amount of potential complications resulting from immune rejection, graft-versus-host-diseases, the duration of engraftment and infectious complications. The patient would benefit significantly because, if engraftment and reconstitution of the hematopoietic system does not occur after transplantation, the physician can rapidly detect this rejection and proceed with a second transplant

To realize the promise of stem cell based therapy, a number of significant hurdles must be surmounted. Through research, we have provided solutions to each of these hurdles as listed below.

1. To be useful for transplant purposes, stem cells must be reproducibly made to:

(a) Be of sufficient quantity to be useful for transplantation. In the prior art, having sufficient stem cells required stem cells to proliferate extensively *in vitro. In vitro* passage and proliferation of cells can lead to neoplastic transformation or the loss ofpluripotency. The methods of the invention have solved this problem and the need for *in vitro* proliferation by eliminating the *in vitro* proliferation step. The solution is to collect a sufficient quantity of stem cells to allow transplantation through the use of primary and unamplified cells only - using the novel methods of the invention. Primary cells refers to cells that are collected from a subject and which has not undergone significant divisions *in vitro.* In a preferred embodiment, the primary cells have not undergone any proliferation *in vitro.* This is possible, for example, if the cells collected from a subject are preserved immediately or preserved immediately after processing. In other words, the cells are not cultured *in vitro.* For example, using the methods of the invention, stem cells may be collected over the course of years and multiple collection sessions ensuring an almost unlimited amount of stem cells can be collected. In fact, the number of stem cells collected is only limited by a patient's willingness to endure multiple collections. However, even this problem may be surmounted because patients in high risk groups would have more motivation to bank stem cells.

2. Stem cells must survive in the recipient after transplant. Our unique method of autologous transplantation (*e.g*., following long term cell preservation or cryopreservation) has eliminated the risk that the transplanted stem cells would be killed by host rejection. In addition, current cryogenic techniques ensure cell survival rates of over 95%. Thus, the risk of low cell survival has been significantly reduced.

3. For successful therapy, stem cells must integrate into the surrounding tissue after transplant. Since the methods of the invention involve autologous transfer, the transplanted cells and the patient's own cells are extremely compatible and integration is not a problem.

4. For long term treatment, the transplanted stem cells must function appropriately for the duration of the recipient's life. Once again, autologous transplanted stem cells would be expected to survive as long as the patient's own stem cells since they have identical phenotypes and genotypes.

5. Transplanted stem cells must avoid harming the recipient in any way. Autologous transplant methods of this invention absolutely eliminate the possibility of graft vs. host disease.

To summarize, the promise of stem cell therapies is an exciting one, but significant technical hurdles remain for its practical use. However, for reasons stated above, we have overcome these hurdles by using the methods of this invention.

The present invention presents methods for using autologous stem cell transplants, such as those from peripheral blood, and bone marrow from post-birth human (including baby, child and adult), for the treatment of diseases. In a non-limiting example of the invention, the diseases treated are cancer and immunodiseases such as acquired immunodeficiency syndrome (AIDS). The invention has an advantage over umbilical cord blood transplants since for the overwhelming majority of children and adult, their umbilical cord blood at birth is no longer available.

While the use of stem cells for treatment of myelodeficiency and immunodeficiency shows promise, delayed recovery of a patients cellular and organ systems (such as the hematopoietic system, the hepatitic system, the immune system) is often a byproduct of insufficient stem cell dose. The recovery of the organ system is delayed because a low stem cell dose requires a longer period to reconstitute a compromised or damaged organ system. This delayed recovery remains an important source of morbidity and motility for many transplant patients. The presence of progenitor cells, partially and possibly, terminally differentiated cells, may have significant advantages with respect a shorter time to reconstitution and other physiological benefits in the post-infusion period.

Other advantages of the present invention include, at least, the following:

(1) Since the method uses autologous transplant, there is no need to expend time, money and energy in "cleansing" the SC (stem cell(s)) for subject marrow of potentially dangerous mature T cells (thought to be important for the development of graft-versus-host-disease). Examples of cleansing process are: chemicals or monoclonal antibody (OKT3) that specifically recognizes and eliminate mature T cells, filtration, column chromatography or batch chromatography. It should be noted that chromatography may involve affinity chromatography using antibodies specific for stem cells, progenitor cells, or terminally differentiated cells. In the case of patients with certain existing diseases other than the targeted disease at the time of collection, the "healthy" harvested cells will not contain the disease vectors of the targeted disease, such as in the case of AIDS patients, the pre-disease cells will not contain the AIDS virus.

(2) Since the method uses autologous transplant, there is no need to laboriously locate SC or bone marrow from another subject or to conduct tests to ensure that the stem cell (SC) or bone marrow matches that of the recipient. Further, there is no fear that matching SC or bone marrow may not be found or that crucial time is lost to test and locate such matching SC or bone marrow such that the recipient may be in mortal danger or incur fatal injury by the time a match is found.

(3) Since the method uses autologous transplant, there is no fear of graft-versus-host disease; or immune rejection.

(4) Autologous stem cells do not carry the risk of infectious disease found when one person's stem cells are used for another person.

(5) Since the method uses autologous transplant, there is no fear of transplant transmitted disease (*e.g*. HIV, CMV, hepatitis, syphilis etc.)

(6) Of particular importance is the fact that these autologous transplants are harvested prior to the development of disease (pre-disease stage) as compared to the period of time after disease occurs (post-disease stage). The pre-disease state autologous stem cell transplant has the following advantages over transplantation using autologous stem cells harvested after disease occurs:

(i) The previously harvested pre-disease cells will be younger. Among the possible advantages associated with youth are: the cells will likely to be more resilient, more versatile, and would retain normal (or relative more normal) activities and a full range of (or broader range of) activities, and thus more well-equipped and more vigorous in combating a disease, as compared to stem cells collected after disease occurs. Further, due to advance in age (*e.g*., in old age), certain population of cells may be depleted, missing or no longer be available for harvesting at a later stage in a human's life. Also, certain cellular functions or genes may be turned off in older cells, down-regulated, or lost, due to the natural aging process, aged related deterioration, mutation, or accumulated "wear-and-tear", or environmental assaults over the years, etc. Further, older cells (post-disease versus pre-disease cells) may contain more mutations, defects, due to age or mistakes in the replication process, or environment assaults.

(ii) The previously harvested (pre-disease) cells from the donor, prior to him becoming a patient, will be healthy cells (or healthier than the current cells existing in the patient) and may be more easily grown or programmed, or more readily programmable than the post-disease cells.

(iii) The pre-disease population of the harvested cells will be healthy or will contain more healthier (or less diseased) cells than the post-disease population of cells, and thus the population of the pre-disease harvested cells will not be contaminated or be less contaminated by diseased cells which may be reintroduced into the patient and potentially cause a relapse.

For example, in the case of cancer treatment, the present invention has the following advantages over the prior art described above. The infused cells will not be contaminated with cancer cells (or will be less contaminated with cancer cells if the collection occurred after the disease has taken hold but before its diagnosis) as compared to the cells collected from a patient who has already developed cancer. Therefore, no laborious, time-consuming, inefficient methods (that may even inadvertently introduce undesirable chemicals) assays and screenings are required to cleanse the harvested cell population to remove cancer cells from the pre-disease harvested cells. Further, the present invention eliminates or reduces the possibility of causing a relapse through infusion of sub-optimal cancer cell depleted stem cell products.

(iv) The population of harvested cells may be more "well-rounded" or more normal/healthy in that a full range of normally occurring cells will be present relative to older diseased cell population. For example, the peripheral blood SC collected from an AIDS patient will be deficient in T helper cells which are decimated by the AIDS virus; but found in normal number in the previously collected and healthy population of cells.

(v) In an optional step, the collected stem cells, progenitor cells and terminally differentiated cells can be multiplied in culture. The multiplication may be performed before or after cryopreservation. Using this method, the number of stem cells available for therapy may be expanded. Thus, the population of healthy cells may be increased by cellular expansion, and infused into the patient to greatly boost his immunodefense in the number of cells available and that the cells are healthy.

(vi) Furthermore, processed stem cells may undergo immune modulation or cellular adaptation inherent in the processing and cryopreservation technique which may improve the stem cell product.

Although the foregoing invention has been described in some detail by way of illustration and example for purposes of clarity and understanding, the above are by way of example, and are not meant to be limiting. It will be obvious that various modifications and changes that are within the skill of those skilled in the art are considered to fall within the scope of the appended claims.

Future technological advancements that allow for obvious changes in the basic invention herein are also within the claims.

All publications, figures, patents and patent applications cited herein are hereby expressly and fully incorporated by reference herein for all purposes to the same extent as if each had been fully set forth herein.

**Characterization Of Peripheral Blood Stem Cells**

One aspect of the invention is directed to the use of mobilized peripheral blood stem cells as a diverse population of stem cells. These stem cells can be characterized by phenotype or genotype using a number of techniques. For example, phenotype based characterization can involve detection of cell surface antigen by using a fluorescent activated cell sorter. Cell surface antigens that can be detected include CD34, KDR and CD133. Other detection can involve, for example, review of chromosome structure during metaphase by karyotyping. Genotype characterization can involve one of more of the following techniques: isolation of nucleic acids (DNA, RNA), electrophoresis, PCR, gene chip analysis, and genomic sequencing. Furthermore, the proteome of the cells can be analyzed by protein chips, one dimension and two dimensional gel electrophoresis, and column chromatography.

A review of the phenotype and genotype of peripheral blood stem cells can provide diagnostic and prognostic purposes. For example, it has been suggested that there is a close interplay between the presence of endothelial progenitor cells and cardiovascular risk factors. That is, the presence of higher amounts of endothelial stem cells in the blood cells is correlated with a good outcome (less risk of cardiovascular events). Cardiovascular events that can be predicted includes arterial hypertension, hyperlipidemia, diabetes, coronary artery diseases, myocardial infarctions, major cardiac events, need for revascularization, need for hospitalization, stroke, or death from cardiovascular events, and a combination of these indications.

Another aspect of the invention is directed to the collection of neural stem cells, mesenchymal stem cells, or pancreatic stem cells from the peripheral blood. The peripheral blood cells can be sorted, for example, by FACS, to isolate cells which displays phenotype typical of neuronal stem cells, mesenchymal stem cells, or pancreatic stem cells. This sorting can be performed to isolate stem cell displaying cell surface receptors typical of these cell lineages. This technique can be expanded to isolate stem cells from other organs.

The markers on the peripheral blood cells can be sorted simultaneously or serially. For example, the cells can be sorted for one marker on a FACS and collected. The collected cells can be sorted for a second marker on a FACS also. Alternatively, the cells can be sorted with both markers simultaneously.

It is understood that FACS can involve the binding of antibodies (or derivatives thereof) which is specific for a cell surface marker.

Another aspect of this application is directed to the collection of stem cells from the peripheral blood of a patient at different stages of the patient's life. The constitution/distribution of the stem cell is determined by FACS, phenotype or genotype. That is, the amount of each stem cell type and the quality of each type of stem cells type can be measured using any method described in this specification and collected and stored. Furthermore, the stem cells collected can be cyropreserved. This data will provide a snapshot of the stem cell constitution/distribution and can serve as valuable data for diagnostic and prognostic applications. That is, the collective set of stem cell markers represents an important fingerprint of the current and potentially future health of an individual and can be of prognostic value. This collected stem cell population can be compared to another collected stem cell population collected from the same individual at a different date to see if the constitution/distribution the stem cells have changed. Any difference can also be noted and can have diagnostic value.

The markers on the peripheral blood stem cells to be detected and analyzed by any of the methods of the invention include markers of T-regulatory cells that can be therapeutic value for the treatment of auto-immune disease, viral disease, graft verses host disease and cancer.

Additional methods not described are well known to one of skill in the art or are described in publications such as published U.S. patent applications US20040258673(A1) or US20040265281(A1), hereby incorporated by reference in their entireties.

According to preferred embodiments, the mobilized peripheral blood stem cells of the present invention may be used as a diverse population of stem cells and progenitor cells that can be phenotypically characterized by FACS, immunofluorescence, PCR and other methods for their ability to become committed linear specific progenitor cells. Stem cell markers may be used to phenotypically characterize the peripheral blood stem cells. These stem cell markers, particularly in the field of cardiovascular disease, can be predictive of future cardiac events including myocardial infarction, a major cardiac event, hospitalization and death. Such markers include CD34 and CD133 as markers for endothelial progenitor cells. These markers also include markers of T-regulatory cells that can be therapeutic value for the treatment of auto-immune disease, viral disease, graft verses host disease and cancer.

These stem cell markers can may also be used to identify and isolate neuronal progenitor cells, mesenchymal stem cells, pancreatic stem cells, or stem cells for any organ in the body. These markers may also be used serially in a patient in order to optimize "stem cell" health for a future stem cell harvest.

This collective set of stem cell markers represents an important fingerprint of the current and potentially future health of an individual and may be of prognostic value. For example, this collection of stem cell markers may serve as biomarkers for the breadth of the stem cell population to become different lineage specific progenitor cells.

***References***

1. C. Sonnenschein, A. M. Soto, The Society of Cells-Cancer And Control of Cell Proliferation, BIOS Scientific Publishers Ltd and Springer-Verlag, New York, 1999.

2. G. B. Pierce et al. Cancer-A Problem In Developmental Biology. Prentice Hall, New York, 1974, pp. 79-84; G. B. Pierce, in The Biological Basis of Cancer, R. G. McKinnel, R. E. Parchment, A. O. Perantoni, G. B. Pierce, Eds., Cambridge Univ. Press, Cambridge UK, 1998, pp. 39-47.

3. United States Patent Application, Publication No. 2001 0000204 A1, of Castino et al., published Apr. 12, 2001. Hereinafter referred to as "Castino *et al.*

4. Gudemann, C., Wiesel, M. And Staehler, G., Intraoperative Autotransfusion In Urologic Cancer Surgery By Using Membrane Filters, XXIIIrd Congress of the ISBT, abstracts in Vox Sang., 67 (S2), 22.).

5. Miller, G. V., Ramsden, C. W. and Primrose, J. N., Autologous transfusion: an alternative to transfusion with banked blood during surgery for cancer, B. J. Surg. 1991, Vol. 78, June, 713-715.

6. Douay et al., 1986, Recovery of CFU-GM from cryopreserved marrow and in vivo evaluation after autologous bone marrow transplantation are predictive of engraftment. Exp Hematol. 14(5):358-365; Knight, 1980, Preservation of Leukocytes in Low Temperature Preservation in Medicine and Biology, Ch. 6, Ashwood-Smith and Farrant (University Park Press, Baltimore) pp. 121-137.

7. United States Patent Application Publication No. 20020146680 A1, of Rich, Ivan N., published Oct. 10, 2002, entitled "High-throughput stem cell assay of hematopoietic stem and progenitor cell proliferation, incorporated herein by reference in its entirety.

8. United States Patent Application Serial No. 10/819,342, filed April 5, 2004 and United States Patent Application Serial No. 60/460,362, filed April 3, 2003, incorporated herein by reference in their entireties.

The following examples are illustrative, but not limiting, of the methods and compositions of the present invention. Other suitable modifications and adaptations of the variety of conditions and parameters normally encountered in therapy and that are obvious to those skilled in the art are within the spirit and scope of the embodiments.

***Example 1: Collection and Cyropreservation of peripheral blood stem cells***

Peripheral blood stem cells were collected from a patient who elected to store his stem cells for future autologous transfer. Briefly, the patient's vital medical statistics including age (45 years) weight (150 pound), sex (male), blood pressure (120/80) and hemocrit value (42%) were collected. The total amount of blood was calculated from the sex, height and weight of the donor, and the process volume of whole blood per cycle was determined from the hematocrit value. The apheresis machine was set with the speed of rotation of the centrifuge at 4800 rpm and the critical flow at 50 ml/min.

Stem cells were collected from the patient for 3 hours. The total volume of collected cells was 250ml. These cells were washed in sterile RPMI media and resuspended in cryopreservation media (RPMI + 10% glycerol + 5% DMSO). Half the cells were aliquoted at approximately 10 million cells/ml in 20 cryovials from Corning. The other half were placed in a cryobag from Baxter.

Cyropreservation of the stem cells was performed in a MVE CRF 2000 model Control Rate Freezer with the following programmed temperature ramp: cool the freezing chamber to 4°C; decrease the temperature from 4°C to -11° c at a rate of -0.7 °C /min; decrease the temperature from -11° c to -60°C at a rate of -25°C /min; hold the temperature at -60°C for 1 minute 30 seconds; increase the temperature from-60°C to -25°C at a rate of +10°C/min; decrease the temperature from -25°C to -45°C at a rate of 0.7°C/min; decrease the temperature from -45°C to -60°C at a rate of -5 °C/min; decrease the temperature from -60°C to -95 °C at a rate of -10 °C/min; and hold at -95 °C for 10 min. The cells are then transferred to liquid nitrogen storage tank for a quarantine period until all the laboratory results are received and transferred to a permanent storage in liquid nitrogen vapor.

***Example 2 Viability Testing of Cyropreserved Cells***

Chilled vials of cells were removed from liquid nitrogen storage and immersed in 37°C water bath. Then the cells were centrifuged at 1000 rpm for 5 minutes. Supernatant was aspirated off and cells were washed twice with PBS.

After washing, the cells were reconstituted in physiological saline solution. An aliquot was taken and stained with trypan blue dye which only binds dead cells. Counting the stained cells on a hemocytometer showed that greater than 95% of the cells are viable.

The cells, after thawing, will be injected into patients in an autologous transfer.

***Example 3***

Peripheral blood stem cells (PBSC) was collected from non-disease or pre-disease donors according to a preferred embodiment disclosed herein. Figures 4 and 5 provide a general outline for the procurement, collection, and procession of donor PBSCs. Donor eligibility was defined and selected in accordance with Reference Standard 5R-A (AABB CT Standards, 2004 edition pp 42 - 44). Donors are adults 18 years of age or older. The usual minimum body weight for donor is 110 pounds although the process may be adjusted for patients of lower weight. Donors are additionally screened for abnormal results on medical history screening or testing that may affect the recipient's health or the therapeutic value of the PBSC product. Testing to identify potential for disease transmission is performed prior to collecting the PBSC product under the following considerations:

| Laboratory Tests & Results: | Preferred but not absolute disposition |
|---|---|
| HIV (Human immunodeficiency virus) | Positive Not acceptable for donation |
| HBsAg (Hepatitis B virus surface antigen | Positive Not acceptable for donation |
| HCV (Hepatitis C virus) | |
| HTLV (Human T cell lymphoma virus) | Positive Not acceptable for donation |
| HBcore antibody Hepatitis B antibody) | Positive Is acceptable for donation |
| RPR (Syphilis-Treponema pallidum) | Positive Is acceptable for donation |
| Cytomegalovirus | Positive Is acceptable for donation |
| ABO/Rh (Blood type) | Not applicable |
| CBC (Complete Blood count) | Physicians must assess abnormalities |
| Hct (Hematocrit) | <33% unacceptable, deferred patient |
| | may be treated to increase Hb. |
| HLA (tissue transplantation antigens) | Not applicable |

PBSCs were collected from donors over a three day course. Here, a first dose of 480 µg of G-CSF is administered by subcutaneous injection to a pre-disease subject on Day 1. This was followed on Day 2 by a second dose of 480 µg of G-CSF by subcutaneous injection. On Day 3, blood is drawn from one arm of the donor and enters the apheresis instrument where the stem cells are separated and collected. The rest of the whole blood is then returned to the donor.

The administration of 480 µg of G-CSF via subcutaneous injection over two consecutive days resulted in an increase in the peripheral count of total nucleated cells from a mean of 169.4 X 10⁸/L (n=21) in unstimulated control to a mean of 252.3 X 10⁸/L (n=22). The absolute number of CD34+ cells increased from a mean of 64.29 X 10⁶/L (n=21) to 85.4 X 10⁶/L (n=22). The results of obtained for G-CSF stimulated donors is presented in the table below.

| **G-CSF Stimulated Donors** | | | | |
|---|---|---|---|---|
| **ID Number** | **Weight/Kg.** | **Total TNC x10e8** | **Total CD34+ x10e6** | **Percent CD34+** |
| A005 | 53.6 | 92.4 | 66.53 | 0.72 |
| A006 | 61.4 | 98.9 | 162.20 | 1.64 |
| A007 | 77.7 | 305.0 | 48.80 | 0.16 |
| A008 | 68.2 | 282.0 | 5.64 | 0.02 |
| A009 | 95.5 | 245.5 | 144.85 | 0.59 |
| A010 | 81.8 | 348.0 | 20.88 | 0.06 |
| A011 | 77.3 | 344.1 | 120.44 | 0.35 |
| A012 | 81.8 | 140.8 | 16.90 | 0.12 |
| A013 | 86.4 | 313.7 | 98.82 | 0.06 |
| A014 | 102.7 | 208.8 | 123.19 | 0.59 |
| A015 | 70.5 | 136.0 | 63.92 | 0.47 |
| A016 | 104.1 | 226.0 | 131.08 | 0.58 |
| A017 | 60.0 | 222.0 | 66.60 | 0.30 |
| A018 | 83.6 | 141.8 | 5.67 | 0.04 |
| A019 | 111.4 | 187.8 | 180.29 | 0.96 |
| A020 | 81.8 | 259.7 | 83.10 | 0.32 |
| A021 | 88.6 | 225.3 | 148.70 | 0.66 |
| A022 | 88.6 | 262.1 | 20.97 | 0.08 |
| A023 | 83.6 | 353.3 | 105.99 | 0.30 |
| A024 | 52.3 | 234.9 | 46.98 | 0.20 |
| A027 | - | 327.0 | 176.58 | 0.54 |
| A030 | 101.8 | 602.0 | 120.40 | 0.20 |

Peripheral Blood units that do not meet one or more of the acceptable criteria will preferably be discarded. A number of predetermined tests, standards, and criteria are used to determine the potential suitability of a Peripheral Blood unit for processing, cryopreservation, and ultimate transplant. Peripheral blood stem cells (PBSC) may be stored in liquid nitrogen (vapor or liquid phase) long term. The volume of product in the bag is preferrably at least 100 ml. The WBC count on the PBSC product is preferred to be at least 5 x 10⁶/ml, although values higher or lower than this number is still useful.

While the invention has been described with reference to particularly preferred embodiments and examples, those skilled in the art recognize that various modifications may be made to the invention without departing from the spirit and scope thereof.

All of the above U.S. patents, U.S. patent application publications, U.S. patent applications, foreign patents, foreign patent applications and non-patent publications referred to in this specification and/or listed in the Application Data Sheet are incorporated herein by reference, in their entirety.
In view of the foregoing, it will be appreciated that the invention described herein *inter alia* relates to the following items:
1. A method of collecting autologous adult stem cells from a pre-disease subject comprising the steps of:
   administering to the pre-disease subject at least two doses of G-CSF of about 1 µg/kg/day to 8 µg/kg/day;
   collecting adult stem cells from peripheral blood pre-disease subject using an apheresis process;
   at the time of collection, earmarking the collected cells for use by the subject; and
   preserving the collected cells to maintain the cellular integrity of the cells.
2. The method of item 1, wherein the pre-disease subject is administered at least two doses of G-CSF within a 2 to 6 day period.
3. The method of item 2, wherein the at least two doses of G-CSF is administered on two consecutive days, with the subject receiving one dose per day.
4. The method of item 3, wherein the subject receives two doses of G-CSF administered on consecutive days.
5. The method of item 7, wherein the collection of adult stem cells from peripheral blood using an apheresis process is conducted the day after the second dose of G-CSF is administered.
6. The method of item 4, wherein the G-CSF is administered subcutaneously.
7. The method of item 5, wherein about 480 µg per dose of G-CSF is administered subcutaneously to the subject.
8. The method of item 1, wherein the pre-disease subject is administered at least two doses of G-CSF within about 12 to about 36 hours of each other.
9. The method of item 7, wherein the collection of adult stem cells from peripheral blood using an apheresis process is conducted about 12 to about 36 hours after the second dose ofG-CSF is administered.
10. The method of item 9, wherein the G-CSF is administered subcutaneously.
11. The method of item 10, wherein about 480 µg per dose of G-CSF is administered subcutaneously to the subject.
12. The method of item 3, wherein the G-CSF is administered to a subject at a dose of about 4 to about 6 µg/kg/day or equivalent thereof.
13. The method of item 3, wherein about 50 µg to about 800 µg per dose of G-CSF is administered subcutaneously to the subject.
14. The method of item 3, wherein about 300 µg to about 500 µg per dose of G-CSF is administered subcutaneously to the subject.
15. The method of item 1, wherein the subject is a human subject that has met at least one condition selected from the group consisting of between 10 and 200 kg in weight and between 2 to 80 years old.
16. The method of item 1, wherein the collecting step is conducted when the subject is an adult or a non-neonate.
17. The process of item 1, wherein the collecting step includes the step of collecting at least greater than 100 X 10⁸ total nucleated cells per subject in a single collection process.
18. The process of item 1, wherein the collecting step includes the step of collecting at least greater than 250 X 10⁸ total nucleated cells per subject in a single collection process.
19. The process of item 17, wherein the collecting step is undertaken over multiple sessions.
20. The process of item 1, wherein the preserving step comprises storing the collected cells in a stem cell bank.
21. A process of stem cell banking comprising the steps of:
   (a) administrating one or more stem cell potentiating agents to a person to increase the amount of stem cells in the peripheral blood of said person;
   (b) collecting at least one population of stem cells and at least one population of non-stem cells from peripheral blood of said person using an apheresis process, wherein said person has no immediate perceived health condition requiring treatment using his own collected stem cells;
   (c) preserving the at least one population of stem cells and the at least one population of non-stem cells as a preserved populations of cells;
   (d) retrieving the preserved populations of cells for autologous transplantation of the at least one population of stem cells and at least one population of non-stem cells into the person.
22. The process of item 21 wherein said one or more stem cell potentiating agents is selected from the group consisting of G-CSF, GM-CSF, dexamethazone, a CXCR4 receptors inhibitor and a combination thereof.
23. The process of item 22 wherein the CXCR4 receptor inhibitor is selected from the group consisting of AMD3100, ALX40-4C, T22, T134, T140, and TAK-779.
24. The process of item 21 wherein said administration is performed for at least one week before said collecting step.
25. The process of item 21 wherein said health condition is selected from the group consisting of a neoplastic disorder, an immune disorder, and leucopenia.
26. The process of item 21, wherein the collecting step is conducted when the person is an adult or a non-neonate child.
27. The process of item 21, wherein the collecting step is performed at least two times.
28. The process of item 21, wherein the collecting step is performed at least three times.
29. The process of item 21, wherein the collecting step is performed at least five times.
30. The process of item 21, wherein the collecting step collects at least 1 x 10⁶ total nucleated cells per kilogram weight of the person in a single collection session.
31. The process of item 21, wherein the collecting step collects at least 2 x 10⁶ total nucleated cells per kilogram weight of the person in one or more collection session.
32. The process of item 21, wherein the collecting step collects at least 3 x 10⁶ total nucleated cells per kilogram weight of the person in one or more collection session.
33. The process of item 21, wherein the collecting step collects at least 5 x 10⁶ total nucleated cells per kilogram weight of the person in one or more collection session.
34. The process of item 21 wherein said apheresis process is performed for at least one hour in said collecting step.
35. The process of item 21 wherein said apheresis process is performed for at least two hours in said collecting step..
36. The process of item 21 wherein said apheresis process is performed for at least three hours in said collecting step.
37. The process of item 21 wherein said apheresis process is performed for at least four hours in said collecting step..
38. The process of item 21 wherein the apheresisprocess releases additional cells into the peripheral blood of said person.
39. The process of item 38 wherein said additional cells are selected from the group of stem cells, progenitor cells, and terminally differentiated cells.
40. The process of item 21 wherein said preserving step preserves cells collected in said collecting step before substantial cell divisions.
41. The process of item 21, wherein the preserving step comprises the step of processing the stem cells into multiple separate containers for storage.
42. The process of item 41 wherein the processing step comprises the step of isolating one cell population enriched or depleted for a stem cell surface antigen.
43. The process of item 42 wherein said stem cell surface antigen is selected from the group consisting of CD34, KDR, CD45, and CD 133.
44. The process of item 41 wherein the processing step comprises the step of isolating one cell population enriched or depleted for a progenitor cell surface antigen.
45. The process of item 44 wherein said progenitor cell surface antigen is selected from the group consisting of CD45, Lin, Muc-18, CK19, Nestin, and KDR.
46. The process of item 41 wherein the processing step comprises the step of isolating one cell population enriched for one or more terminally differentiated cell surface antigen.
47. The process of item 46, wherein said one or more terminally differentiated cell surface antigen are selected from the group consisting of CD-4, CD-8, Flk1, myosin, bone specific alkaline phosphatase, osteocalcin, bone morphogenic protein receptor, CD38, CD44, Thy-1, and adipocyte lipid binding protein.
48. The process of item 41 wherein said processing step comprises the step of analyzing at least one characteristic of one cell in said one population of stem cells or at least one population of non-stem cells.
49. The process of item 48 wherein said at least one characteristic is a DNA or RNA sequence of said cell.
50. The process of item 48 wherein said at least one characteristic is a proteome of said cell.
51. The method of item 41, wherein the processing step involves treating said one population of stem cells or said at least one population of non-stem cells with an agent to enhance the storage, viability, or therapeutic ability of said cell population.
52. The process of item 51 wherein said treating is transforming said one population of stem cells or said at least one population of non-stem cells with a nucleic acid.
53. The process of item 21, wherein the process is performed without HLA typing of said one population of stem cells or said at least one population of non-stem cells
54. The process of item 21, wherein the preserving step comprises the step of determining from the collected population of cells at least a distinctive property associated with the person prior to storing in a the stem cell bank, so as to provide a means of secured identification to match the collected stem cells with the person at the time of use.
55. The process of item 54 wherein said distinctive property is a DNA or RNA sequence.
56. The process of item 54 wherein said distinctive property is a proteome of a cell said one population of stem cells or said at least one population of non-stem cells.
57. The process of item 54, wherein the determining step further includes providing an indicia with each population of cells representing information of said distinctive property
58. The process of item 57, wherein the indicia is embodied in at least one of a label, bar code, magnetic strip, and microchip.
59. The process of item 57 wherein the indicia is embedded within the preserved collected populations of cells.
60. The process of item 21, wherein said preserving step comprises cryopreservation of said at least one population of stem cells and at least one population of non-stem cells.
61. The process of item 60 wherein said at least one population of stem cells and at least one population of non-stem cells are cryopreserved in separate containers.
62. The process of item 60 wherein said at least one population of stem cells and at least one population of non-stem cells are cryopreserved in the same container.
63. The process of item 21, further comprising the step of:
   (f) administering said stem cell to said person in an autologous transfer.
64. The method of item 63 wherein said method is used to treat a person in a leukopenic state.
65. The process of item 63 wherein said autologous transfer is performed without HLA typing.
66. A cellular therapy product comprising an autologous mixture of peripheral blood stem cells and non-stem cells, wherein the non-stem cells comprise progenitor cells and optionally functional cells.
67. The cellular therapy product of item 67 comprising from about 10% to about 90% peripheral blood stem cells and from about 10% to about 90% non-stem cells.
68. The cellular therapy product of item 67 comprising from about 10% to about 80% peripheral blood stem cells and from about 20% to about 90% non-stem cells.
69. The cellular therapy product of item 67 comprising from about 10% to about 60% peripheral blood stem cells and from about 40% to about 90% non-stem cells.
70. The cellular therapy product of item 67, wherein the non-stem cells are selected from the group consisting of hematopoietic progenitor cells, neural progenitor cells, glial progenitor cells, oligodendrocyte progenitor cells, skin progenitor cells, hepatic progenitor cells, muscle progenitor cells, bone progenitor cells, mesenchymal stem or progenitor cells, pancreatic progenitor cells, progenitor chondrocytes, stromal progenitor cells, cultured expanded stem or progenitor cells, cultured differentiated stem or progenitor cells, or combinations thereof.
71. The cellular therapy product of item 67, wherein the functional cells are selected from the group consisting of terminally differentiated hematopoietic cells, terminally differentiated neural cells, terminally differentiated glial cells, terminally differentiated oligodendrocytes, terminally differentiated skin cells, terminally differentiated hepatic cells, terminally differentiated muscle cells, terminally differentiated bone cells, terminally differentiated adipocytes, terminally differentiated pancreatic cells, chondrocytes, stromal cells, cultured differentiated stem or progenitor cells, or combinations thereof
72. A method of enhancing the engraftment of stem or progenitor cells comprising administering to a subject an autologous mixture ofperipheral blood stem cells, progenitor cells, and optionally functional cells.

## Claims

1. A cellular therapy product comprising an autologous mixture of peripheral blood stem cells and non-stem cells, wherein the non-stem cells comprise progenitor cells and optionally functional cells.

2. The cellular therapy product of claim 1, comprising from about 10% to about 90% peripheral blood stem cells and from about 10% to about 90% non-stem cells, or comprising from about 10% to about 80% peripheral blood stem cells and from about 20% to about 90% non-stem cells, or comprising from about 10% to about 60% peripheral blood stem cells and from about 40% to about 90% non-stem cells.

3. The cellular therapy product of claim 1, wherein the non-stem cells are selected from the group consisting of hematopoietic progenitor cells, neural progenitor cells, glial progenitor cells, oligodendrocyte progenitor cells, skin progenitor cells, hepatic progenitor cells, muscle progenitor cells, bone progenitor cells, mesenchymal stem or progenitor cells, pancreatic progenitor cells, progenitor chondrocytes, stromal progenitor cells, cultured expanded stem or progenitor cells, cultured differentiated stem or progenitor cells, or combinations thereof.

4. The cellular therapy product of claim 1, wherein the functional cells are selected from the group consisting of terminally differentiated hematopoietic cells, terminally differentiated neural cells, terminally differentiated glial cells, terminally differentiated oligodendrocytes, terminally differentiated skin cells, terminally differentiated hepatic cells, terminally differentiated muscle cells, terminally differentiated bone cells, terminally differentiated adipocytes, terminally differentiated pancreatic cells, chondrocytes, stromal cells, cultured differentiated stem or progenitor cells, or combinations thereof.

5. The cellular therapy product of claim 1, which is made by collecting adult stem cells from peripheral blood of a pre-disease subject using an apheresis process; wherein the pre-disease subject was administered one or more stem cell potentiating agents.

6. The cellular therapy product of claim 5, wherein the stem cell potentiating agent is G-CSF, GM-CSF, dexamethasone, or a CXCR4 receptor inhibitor.

7. The cellular therapy product of claim 5, wherein the pre-disease subject was administered at least two doses of G- CSF of about 1 µg/kg/day to 8 µg/kg/day.

8. The cellular therapy product of claim 5, wherein the pre-disease subject was administered at least two doses of G- CSF of about 4 µg/kg/day to 6 µg/kg/day.

9. The cellular therapy product of claim 5, wherein the pre-disease subject was administered at least two doses of about 480 µg per dose of G-CSF.

10. The cellular therapy product of claim 5, wherein the collection of adult stem cells is conducted the day after the second dose of G-CSF was administered.

11. A method of collecting autologous adult stem cells from a pre-disease subject comprising the steps of:
administering to the pre-disease subject at least two doses of G-CSF of about 1 µg/kg/day to 8 µg/kg/day; and
collecting adult stem cells from peripheral blood of the pre-disease subject using an apheresis process.

12. The method of claim 11, which comprises earmarking the collected cells for use by the subject.

13. The method of claim 11, which comprises preserving the collected cells to maintain the cellular integrity of the cells.

14. The method of claim 11, wherein the pre-disease subject is administered at least two doses of G-CSF within a 2 to 6 day period.

15. The cellular therapy product of any one of claims 7 to 9 or the method of claim12, wherein the at least two doses of G-CSF are administered on two consecutive days, with the subject receiving one dose per day.

16. The method of claim 15, wherein the subject receives two doses of G-CSF administered on consecutive days.

17. The method of claim 11, wherein the collection of adult stem cells from peripheral blood using an apheresis process is conducted the day after the second dose of G-CSF is administered.

18. The method of claim 16, wherein the G-CSF is administered subcutaneously.

19. The method of claim 17, wherein about 480 µg per dose of G-CSF is administered subcutaneously to the subject.

20. The method of claim 11, wherein the pre-disease subject is administered at least two doses of G-CSF within about 12 to about 36 hours of each other.

21. A method of stem cell banking comprising the steps of:
(a) preserving at least one population of cells collected using an apheresis process from the peripheral blood of a subject who has been administered one or more stem cell potentiating agents to increase the amount of stem cells in their peripheral blood, wherein said person has no immediate perceived health condition requiring treatment using their own collected stem cells; and
(b) retrieving the at least one preserved population of cells for autologous transplantation of the at least one population of stem cells into the subject;
wherein said one or more stem cell potentiating agents is selected from the group consisting of G-CSF, GM-CSF, dexamethazone, and a CXCR4 receptor inhibitor.

22. The method of claims 6 or 21, wherein the CXCR4 receptor inhibitor is AMD3100, ALX40-4C, T22, T134, T140, or TAK-779.

23. The method of claim 21, wherein at least 100 X 10⁸ total nucleated cells at least 250 X 10⁸ total nucleated cells are collected from the subject in a single collection process.

24. The cellular therapy product of claim 5 or the method of claim 21,
wherein at least 1 x 10⁶ or at least 2 x 10⁶ or at least 3 x 10⁶ or at least 5 x 10⁶ total nucleated cells per kilogram weight of the person are collected in a single collection process.

25. The cellular therapy product of claim 5 or the method of claims 11 or 21, wherein the preserved cell population is enriched or depleted for a stem cell surface antigen.

26. The cellular therapy product or the method of claim 25, wherein the stem cell surface antigen is CD34, KDR, CD45, CD133, or CXCR4.
